# EUROPEAN PATENT SPECIFICATION

(11) **EP 3 060 576 B1**
(45) Date of publication and mention of the grant of the patent: **26.09.2018**
(21) Application number: 14787180.0
(22) Date of filing: 22.10.2014
(51) Int. Cl.: C12N 9/10, C12N 15/82, C07K 14/415

(54) **NOVEL FIBER-PREFERENTIAL PROMOTER IN COTTON**
NEUARTIGER FASERPRÄFERENTIELLER PROMOTER IN BAUMWOLLE
NOUVEAU PROMOTEUR PRÉFÉRENTIEL DE FIBRE DANS LE COTON

(30) Priority: 24.10.2013 EP 13189991
(43) Date of publication of application: 31.08.2016
(73) Proprietor: Bayer CropScience NV, 1831 Diegem (BE); Commonwealth Scientific and Industrial Research Organisation, Acton ACT 2601 (AU)
(72) Inventor: MEULEWAETER, Frank, B-9820 Merelbeke (BE); LLEWELLYN, Danny J., O'Connor, Australian Capital Territory 2602 (AU)
(74) Representative: Desomer, Jan G.M.
(86) International application number: PCT/EP2014/072678
(87) International publication number: WO 2015/059205

(56) References cited:
- WO-A1-2012/093032
- US-A1- 2013 081 154
- DATABASE Geneseq [Online] 1 January 2004 (2004-01-01), "Cotton fibre transcription factor 4-4 probable promoter cassette #2.", XP002734397, retrieved from EBI accession no. GSN:ADC89724 Database accession no. ADC89724 -& US 2003/106089 A1 (MCBRIDE KEVIN [US] ET AL) 5 June 2003 (2003-06-05) cited in the application
- DATABASE Geneseq [Online] 11 March 1997 (1997-03-11), "Cotton FbLate2-82A gene and promoter.", XP002734398, retrieved from EBI accession no. GSN:AAT43360 Database accession no. AAT43360 -& WO 96/39021 A1 (MONSANTO CO [US]) 12 December 1996 (1996-12-12) cited in the application
- SONG P ET AL: "ISOLATION AND CHARACTERIZATION OF A COTTON FIBER-SPECIFIC PROMOTER", PLANT PHYSIOLOGY, AMERICAN SOCIETY OF PLANT PHYSIOLOGISTS, ROCKVILLE, MD, US, vol. 111, no. 2, SUPPL, 1 January 1996 (1996-01-01), page 55, XP000973716, ISSN: 0032-0889
- KIM H J ET AL: "A NOVEL EXPRESSION ASSAY SYSTEM FOR FIBER SPECIFIC PROMOTERS IN DEVELOPING COTTON FIBERS", PLANT MOLECULAR BIOLOGY REPORTER, SPRINGER SCIENCE+BUSINESS MEDIA B.V, NL, vol. 20, no. 1, 1 March 2000 (2000-03-01), pages 7-18, XP009075630, ISSN: 0735-9640

## Description

### Field of the invention

The present invention relates to materials and methods for the expression of a gene of interest preferentially or selectively in fibers of plants, such as cotton plants. In particular, the invention provides promoters, promoter regions and expression cassettes for regulating fiber-preferential or fiber-selective expression in cotton plants.

### Background of the invention

Cotton fiber is the single most important textile worldwide. About 80 million acres of cotton are harvested annually across the globe. Cotton is the fifth largest crop in the U.S. in terms of acreage production, with an average of 10.3 million acres planted in the years 2006 to 2008. About 90% of cotton grown worldwide is *Gossypium hirsutum* L., whereas *Gossypium barbadense* accounts for about 8%. Consequently, the modification of cotton fiber characteristics to better suit the requirements of the industry and the consumer is a major effort in breeding by either classical methods or by genetically altering the genome of cotton plants. Goals to be achieved include increased lint fiber length, strength, dyability, decreased fuzz fiber production, fiber maturity ratio, immature fiber content, fiber uniformity and micronaire.

Cotton fiber development is a multistage process under the regulation of a vast number of genes, many of which are up-regulated or highly expressed in developing fiber cells (Li, C.H. et al., 2002; Ruan et al., 2003; Wang, S. et al., 2004; Li et al., 2005; Luo et al., 2007).

Each cotton fiber is a differentiated single epidermal cell of the ovule. Approximately half a million fibers are produced per cotton boll, some forming fuzz and some forming lint. Initiation of an epidermal cell into fiber requires a change in cell fate, which is a fundamental biological process involving genetic, physiological and developmental "switches". Genetic mutations, polyploidy, pollination/fertilization, and hormonal regulation can affect the number of cells developing into fibers or alter fiber cell properties (fuzz vs. lint). However, it is unclear how these factors control gene expression changes that orchestrate the pattern and tempo in early stages of fiber development.

In contrast, the morphological development of cotton fibers is well documented in the art. Cotton fibers undergo four overlapping developmental stages: fiber cell initiation, elongation, secondary wall biosynthesis, and maturation. Fiber initiation is a rapid process. The white fluffy fibers begin to develop immediately after anthesis and continue up to 3 days post-anthesis (DPA), which is followed by fiber cell elongation (until 20 DPA). Secondary wall biosynthesis initiates around 15 dpa and continues to 35 DPA, followed by a maturation process until 45-60 DPA. Cotton fibers are derived from ovular epidermal cells (maternal tissues). However, only ∼25-30% of the epidermal cells differentiate into the commercially important lint fibers. The majority of cells does not differentiate into fibers or develop into short fibers or fuzz. For the cells committed to fiber development, cell initiation and elongation are nearly synchronous on each ovule, indicating that changes in gene expression are orchestrated during fiber differentiation and development through intercellular signaling and/or timing mechanisms.

Various promoters driving expression of genes in the cotton seed have been described. Various promoters driving fiber-preferential expression in cotton are also known.

E6 was the first cotton fiber gene identified, and the E6 promoter has been used for engineering cotton fiber quality (John and Keller, 1996). GhRDL1, a gene highly expressed in cotton fiber cells at the elongation stage, encodes a BURP domain containing protein (Li, C.H. et al., 2002), and the GaRDL1 promoter exhibited a trichome-specific activity in transgenic Arabidopsis plants (Wang, S. et al., 2004). GhTUB1 transcripts preferentially accumulate at high levels in fiber, accordingly, the pGhTUB1::GUS fusion gene was expressed at a high level in fiber but at much lower levels in other tissues (Li, X.B. et al., 2002). Promoters of three cotton lipid transfer protein genes, LTP3, LTP6, and FSltp4, were able to direct GUS gene expression in leaf and stem glandular secretory trichomes (GSTs) in transgenic tobacco plants (Hsu et al., 1999; Liu et al., 2000; Delaney et al., 2007), however, they did not exhibit a clear tissue-specificity. For example, in pFSltp4::GUS transgenic tobacco plants, strong GUS activity could be detected in all types of trichomes; in addition, GUS expression was also visible at the leaf margin, vascular tissue, ovules, and root tips (Delaney et al., 2007).

The cotton R2R3 MYB transcription factor GaMYB2 has been shown to be a functional homologue of Arabidopsis GLABRA1 (GL1), a key regulator of Arabidopsis trichome formation. GaMYB2 is expressed in cotton fiber cells at the early developmental stages (Wang, S. et al., 2004). Its promoter drives trichome-specific expression also in Arabidopsis and GST headspecific expression in tobacco (Shangguan et al., 2008).

US patent 7,626,081 discloses a cotton seed-specific promoter found in the alpha globulin gene. The promoter *Gh-sp* is derived from a seed protein gene and is active only in maturing cotton seeds (Song et al., 2000).

US patent application 2003/0106089 discloses a gene expressed in a fiber-specific manner and its promoter.

US patent 6,211,430 discloses a promoter directing expression during late fiber development.

US patent application 20130081154 discloses another promoter directing expressing during late fiber development.

Despite the fact that many promoters known to drive seed-preferential or fiber-preferential expression in cotton plants are available in the art, a need remains for alternative promoters capable of preferential or selective expression in fibers, e.g. for the independent expression of several foreign DNA sequences of interest without the possibility of post-transcriptional silencing due to the use of the same promoter. In addition, the known fiber preferential or fiber selective promoters, each direct a particular temporal, spatial and/or developmental expression pattern, which does not always suit particular goals. There remains thus a need for novel fiber-preferential or fiber-selective promoters with the capacity to control transcription in developing fiber cells, preferably in a more selective manner.

The current invention provides a solution to these problems as described hereinafter in the summary, detailed embodiments, examples, drawings and claims.

### Summary of the invention

The present application discloses an isolated nucleic acid comprising (a) at least about 700 consecutive nucleotides of SEQ ID NO: 1, (b) a nucleic acid having a nucleotide sequence comprising the nucleotide sequence of SEQ ID No 1 from position 1 to position 922, or (c) a nucleotide sequence with at least 95% sequence identity to the nucleotide sequences of (a) or (b), said isolated nucleic acid having fiber-preferential promoter activity in cotton between 15 and 35 days post anthesis (dpa) and not being expressed in 10 dpa fibers and not being longer than 959 nucleotides. Further disclosed herein is a chimeric gene comprising a promoter region as herein described, operably linked to a nucleic acid coding for an expression product of interest, and optionally a transcription termination and polyadenylation sequence. Also disclosed herein are a transgenic plant cell, a transgenic plant and a seed as characterized in the claims. Methods disclosed herein relate to the production of a transgenic plant, effecting fiber-preferential expression of a product in cotton between 15 and 35 days post anthesis (dpa) but not in 10 dpa fibers and of altering fiber properties in a cotton plant as characterized in the claims.

### Brief description of the figures.

Figure 1: Relative expression of Fb8-Like genes in different tissues compared to 35 dpa fibers using the cotton ubiquitin gene as a reference. Primers were to a consensus of a number of very similar Fb8-like ESTs. Values are the average of four technical replicates.
Figure 2: Alignment of deduced protein sequences of the Fb8-like protein encoded by BAC8K12 compared to the cDNA encoded protein from cFB8-like#1 and the FB8-like protein encoded by BAC150F2 (described in US patent application 20130081154) aligned using ClustalW. Identical amino acids are boxed in black and similar amino acids in gray.
Figure 3: A schematic representation of the vector comprising the Fb8-like(8K12) promoter region operably linked to a GUS coding region, which has been used to transform cotton.
Figure 4: Nucleotide sequence of the promoter region of the Fb8-like(8K12) gene in Gossypium hirsutum (SEQ ID No. 1).
Figure 5: 28dpa-fiber GUS staining of Fb8-like(8K12)-GUS bombarded cotton ovule cultures.
Figure 6: GUS staining patterns in different tissues of T0 plants of cotton line T447-22-2 transformed with the Fb8-like(8K12) promoter GUS construct. Intense staining was observed in 20 dpa, but not 10 dpa fibres. Staining was seen in the seedcoat of both 10 and 20 dpa seeds. There was some staining in small glands on leaf petioles (white arrows). Note the absence of any staining of trichomes.
Figure 7: GUS staining around the cut edges of cotyledons of T1 seedling of T447-22-2 expressing the Fb8-like(8K12) promoter GUS construct, but not along edges that haven't been cut. Staining is also evident in the vasculature near cuts and also small glandular trichomes located above the vasculature.
Figure 8: GUS Expression in cotton ovules, seeds and fibres of different ages for transgenic T1 plant 23 of line T447-22-2. Note that GUS staining is strongest in the oldest fibres. There is also some staining of the ovule surface and the seed coat in older seeds.

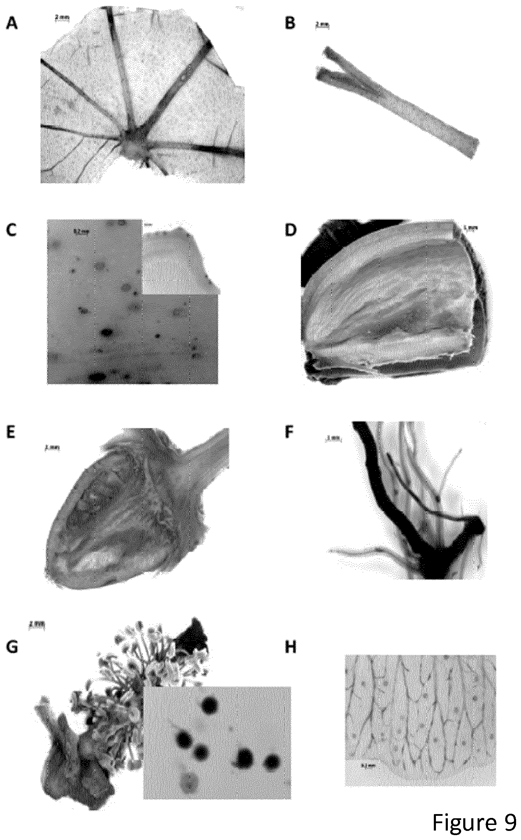

Figure 9: GUS Expression in different cotton tissues from line T447-22-2 T1 plant 23 expressing the Fb8-like(8K12) promoter GUS construct. Panel A) Staining in veins radiating from wounded areas at the base of a mature leaf. Panel B) Staining of the two cut ends of a section of leaf petiole. Panel C) Weak staining of stem surface and in subepidermal glands (inset shows a cross-section of the stem). Panel D) GUS Staining of the cut edges around the septum in a 10 dpa old boll. Panel E) Staining of a sectioned 0 dpa cotton bud with staining at the base where the petals and bracts have been removed and around the cotton ovules. Panel F) Absence of any staining in a root with developing lateral roots. Panel G) GUS staining of the style where petals and bracts have been removed and around the tip of the stigma. Anther walls are unstained and filaments are lightly stained. Note strong staining in segregating pollen grains (cell contents has leaked out during clearing) (inset). Panel H) Staining in the vasculature of the distal end of the petal.
Figure 10: Exhaustion of Acid Orange 7 after dyeing of fibers from a transgenic cotton plant comprising the T-DNA of pTIB359. Light gray bars: fibers from non transgenic control plants; Dark gray bars: transgenic fibers from transgenic cotton lines.

### Detailed description of the invention

### General comments

The sequence listing that is contained in the file named BCS13-2022_ST25.txt, which is 36 kilobytes (measured in MS windows operating system), comprises sequences 1 to 10 and was created on October 21, 2013, is filed herewith.

As used herein, the term "comprising" is to be interpreted as specifying the presence of the stated features, integers, steps or components as referred to, but does not preclude the presence or addition of one or more features, integers, steps or components, or groups thereof. Thus, e.g., a nucleic acid comprising a sequence of nucleotides, may comprise more nucleotides than the actually cited ones, i.e., be embedded in a larger nucleic acid. A chimeric gene as will be described further below which comprises a nucleic acid which is functionally or structurally defined may comprise additional nucleic acids etc. However, in context with the present disclosure, the term "comprising" also includes "consisting of". In other words, the terminology relating to a nucleic acid "comprising" a certain nucleotide sequence, as used throughout the text, refers to a nucleic acid or protein including or containing at least the described sequence, so that other nucleotide or amino acid sequences can be included at the 5' (or N-terminal) and/or 3' (or C-terminal) end, e.g. (the nucleotide sequence of) a selectable marker protein, (the nucleotide sequence of) a transit peptide, and/or a 5' leader sequence or a 3' trailer sequence.

### Nucleic acids

In another aspect, the present application discloses an isolated nucleic acid comprising (a) at least 700 consecutive nucleotides of SEQ ID NO: 1, preferably at least 700 consecutive nucleotides upstream of the nucleotide position 959 in SEQ ID No 1; (b) a nucleotide sequence with at least 95% sequence identity to the nucleotide sequence of (a); (c) a nucleic acid sequence hybridizing under stringent conditions to the nucleotide sequence of (a) or (b); and (d) a nucleic acid sequence complementary to the nucleotide sequence of any one of (a) to (c), wherein said isolated nucleic acid has fiber-preferential promoter activity in cotton between 15 and 35 days post anthesis (dpa) and is not expressed in 10 dpa fibers, and is not longer than 959 nucleotides.

In a particular aspect, the present application discloses the nucleic acid of SEQ ID No. 1 and nucleotide sequences with at least 95% sequence identity to the complete nucleotide sequence of SEQ ID No. 1 which have fiber-preferential promoter activity in cotton between 15 and 35 days post anthesis (dpa) and is not expressed in 10 dpa fibers, and is not longer than 959 nucleotides.

It has been observed that the nucleotide sequence of the fiber-preferential promoter and promoter region of the current invention shares a high degree of nucleotide sequence identity with the fiber-preferential promoter described in US patent application 20130081154, particularly in the region just upstream of the initiation ATG codon, between nucleotide positions 427 and 959 of SEQ ID No. 1. Another region of high nucleotide sequence identity between these two fiber-preferential promoters can be found further upstream (position 1 to 361) separated by a region of little sequence identity. Furthermore, transcription can be initiated at position 922 or position 926 of SEQ ID No. 1.

A fiber-preferential promoter DNA comprising a nucleotide sequence having at least 95%, at least 96%, at least 97%, at least 98%, at least 99% sequence identity with or being identical to the nucleotide sequence of SEQ ID No. 1 between nucleotide position 427 and 922 or having at least 95%, at least 96%, at least 97%, at least 98%, at least 99% sequence identity with or being identical to the nucleotide sequence of SEQ ID No. 1 between nucleotide position 427 and 926, or a fiber-preferential promoter having at least 95%, at least 96%, at least 97%, at least 98%, at least 99% sequence identity with or being identical to the nucleotide sequence of SEQ ID No. 1 between nucleotide position 427 and 959 is also described.

The isolated nucleic acids of this aspect are hereinafter also referred to as "promoter" or "promoter region".

Unless indicated otherwise, the embodiments described below for the promoter sequence disclosed herein are also applicable to respective embodiments of other aspects disclosed herein.

An "isolated nucleic acid" or "isolated nucleic acid sequence", as used in the present application, refers to a nucleic acid as defined above which is not naturally-occurring, such as, for example, an artificial or synthetic nucleic acid with a different nucleotide sequence than the naturally-occurring nucleic acid or a nucleic acid which is shorter than a naturally occurring one, or which is no longer in the natural environment wherein it was originally present, e.g., a nucleic acid coding sequence associated with a heterologous regulatory element such as a bacterial coding sequence operably-linked to a plant-expressible promoter, or in a chimeric gene or a nucleic acid transferred into another host cell, such as a transgenic plant cell.

The length of the isolated nucleic acid sequence of the invention, such as, e.g., a fragment of SEQ ID NO: 1 as disclosed herein, and its position within SEQ ID NO: 1 is to be chosen such that it is sufficiently long, e.g. comprising all elements necessary and sufficient, and positioned such that it is capable of inducing fiber-preferential expression. The promoter regions may be chosen to comprise at least 700 consecutive nucleotides upstream of the nucleotide position 959 in SEQ ID No 1.

Methods of evaluating whether a nucleic acid sequence as described above, which in the present application represents a promoter sequence, is capable of inducing expression of a chimeric gene it is comprised in or, in particular, of a nucleic acid sequence operably linked thereto, in a fiber-preferential manner in cotton between 15 and 35 days post anthesis (dpa) but not in 10 dpa fibers are known to the skilled person.

For example reporter gene studies may be performed in order to evaluate the expression inducing function of a nucleic acid sequence. This includes operably linking the nucleic acid sequence of the invention to a reporter gene such as GUS, introducing the resulting nucleic acid construct in a plant or plant cell, such as in a cotton plant, and evaluating induction of the expression of said reporter gene in different tissues of said plant, as will also be described in more details further below.

The fragment of the nucleic acid sequence described herein which has fiber-preferential promoter activity in cotton between 15 and 35 days post anthesis (dpa) but not in 10 dpa fibers may accordingly comprise at least 700, at least 800, at least 900, at least 950 of SEQ ID NO: 1. In another example, said nucleic acid comprises the nucleic acid sequence of SEQ ID NO: 1. In yet another
example, said promoter sequence consists of the nucleic acid sequence of SEQ ID NO: 1.

However, it will be clear that variants of the nucleic acids herein described, including insertions, deletions and substitutions thereof may also be used to the same effect. Generally, such variants have at least 95%, at least 96%, at least 97%, at least 98% or even at least 99% sequence identity to SEQ ID NO: 1. Such variants should retain their fiber-preferential promoter activity in cotton between 15 and 35 days post anthesis (dpa) but not in 10 dpa fibers, and not be longer than 959 nucleotides..

As used herein, the term "promoter" denotes any nucleic acid sequence, such as a DNA sequence, which is recognized and bound (directly or indirectly) by a DNA-dependent RNA-polymerase during initiation of transcription, resulting in the generation of an RNA molecule that is complementary to the transcribed DNA. This region may also be referred to as a "5' regulatory region". Promoters are usually located upstream of the 5' untranslated region (UTR) preceding the protein coding sequence to be transcribed and have regions that act as binding sites for RNA polymerase II and other proteins such as transcription factors to initiate transcription of an operably linked sequence. Promoters may themselves contain sub-elements (i.e. promoter motifs) such as cis-elements or enhancer domains that regulate the transcription of operably linked genes. The promoter and a connected 5' UTR are also referred to as "promoter region".

A "fiber-preferential" promoter or promoter region in the context of the present invention means that the transcription of a nucleic acid sequence controlled by a promoter is at least 2 times higher, at least 5 times higher, at least 10 times higher, or even 20 times higher in a fiber cell than in cells of another plant tissue such as, for example, leaf tissue. The term does not take into account potential wound-induced expression. In other words, fiber-preferential expression may still be present even if the promoter or promoter regions according to the invention are induced upon wounding of a plant.

The promoter of the present invention may also be fiber specific. A "fiber-specific" promoter means that the transcription of a nucleic acid controlled by said promoter is more than 20 times higher, such as at least 30 times higher, at least 40 times higher, at least 50 times higher
or even 100 times higher in a fiber cell than on average in the cells of other plant tissues at the time of fiber development, such as from 1 dpa until 60 dpa, such as from 10 to 50, from 20 to 50 dpa, from 10 to 35, from 10 to 30 or from 15 to 30 dpa or 15 to 35 dpa. The term does not take into account potential wound-induced expression. In other words, fiber-specific expression may still be present even if the promoters described herein are induced upon wounding of a plant.

Confirmation of promoter activity for a promoter sequence or a functional promoter fragment or promoter region may be determined by those skilled in the art, for example using a promoter-reporter construct comprising the promoter sequence operably linked to an easily scorable marker as herein further explained. The fiber-preferential expression capacity of the identified or generated fragments or variants of the promoter described herein can be conveniently tested by operably linking such nucleic acid sequences to a nucleotide sequence encoding an easily scorable marker, e.g. a beta-glucuronidase gene, introducing such a chimeric gene into a plant and analyzing the expression pattern of the marker in fiber cells as compared to the expression pattern of the marker in other parts of the plant. Other candidates for a marker (or a reporter gene) are chloramphenicol acetyl transferase (CAT), beta-galactosidase (beta-GAL), and proteins with fluorescent or phosphorescent properties, such as green fluorescent protein (GFP) from *Aequora victoria* or luciferase. To confirm promoter function, a nucleic acid sequence representing the promoter is operably linked to the coding sequence of a marker (reporter) gene by recombinant DNA techniques well known in the art. The reporter gene is operably linked downstream of the promoter, so that transcripts initiating at the promoter proceed through the reporter gene. The expression cassette containing the reporter gene under the control of the promoter can be introduced into an appropriate cell type by transformation techniques well known in the art and described elsewhere in this application. To assay for the reporter protein, cell lysates are prepared and appropriate assays, which are well known in the art, for the reporter protein are performed. For example, if CAT were the reporter gene of choice, the lysates from cells transfected with constructs containing CAT under the control of a promoter under study are mixed with isotopically labeled chloramphenicol and acetyl-coenzyme A (acetyl-CoA). The CAT enzyme transfers the acetyl group from acetyl-CoA to the 2- or 3-position of chloramphenicol. The reaction is monitored by thin-layer chromatography, which separates acetylated chloramphenicol from unreacted material.

The reaction products are then visualized by autoradiography. The level of enzyme activity corresponds to the amount of enzyme that was made, which in turn reveals the level of expression and the fiber-preferential functionality of the promoter or fragment or variant thereof. This level of expression can also be compared to other promoters to determine the relative strength of the promoter under study. Once activity and functionality is confirmed, additional mutational and/or deletion analyses may be employed to determine e.g. a minimal promoter region and/or sequences required to initiate transcription. Thus, sequences can be deleted at the 5' end of the promoter region and/or at the 3' end of the promoter region, or within the promoter sequence and/or nucleotide substitutions may be introduced. These constructs are then again introduced into cells and their activity and/or functionality are determined.

Instead of measuring the activity of a reporter enzyme, the transcriptional promoter activity (and functionality) can also be determined by measuring the level of RNA that is produced. This level of RNA, such as mRNA, can be measured either at a single time point or at multiple time points and as such the fold increase can be average fold increase or an extrapolated value derived from experimentally measured values. As it is a comparison of levels, any method that measures mRNA levels can be used. In an example, the tissue or organs compared are a seed or seed tissue such as fibers with a leaf or leaf tissue. In another example, multiple tissues or organs are compared. One example for multiple comparisons is fiber cells compared with 2, 3, 4, or more tissues or organs selected from the group consisting of floral tissue, floral apex, pollen, leaf, embryo, shoot, leaf primordia, shoot apex, root, root tip, vascular tissue and cotyledon. As used herein, examples of plant organs are seed, leaf, root, etc. and example of tissues are leaf primordia, shoot apex, vascular tissue, etc. The activity or strength of a promoter may be measured in terms of the amount of mRNA or protein accumulation it specifically produces, relative to the total amount of mRNA or protein. The promoter expresses an operably linked nucleic acid sequence for example at a level greater than about 0.1%, about 0.2%, greater than about 0.5, 0.6, 0.7, 0.8, or about 0.9%, greater than about 1%, 2%, 3%, 4%, 5%, 6%, 7%, 8%, or about 9%, or greater than about 10% of the total mRNA of the cell it is contained in. Alternatively, the activity or strength of a promoter may be expressed relative to a well-characterized promoter for which transcriptional activity was previously assessed.

In another aspect, fiber-preferential promoters are provided which comprise a nucleic acid sequence having at least 95% , 96%, 97%, 98% or at least 99% sequence identity to SEQ ID NO: 1 or a fragment thereof as defined above (or are identical thereto), such as, for example, a nucleic acid comprising at least 700, at least 800, at least 900, or at least 950 consecutive nucleotides of the nucleotide sequence set forth as SEQ ID NO: 1. Naturally occurring variants of the promoter disclosed herein can be identified with the use of well-known molecular biology techniques, such as, for example, with polymerase chain reaction (PCR) and hybridization techniques as herein outlined before. Such nucleic acid sequences also include synthetically derived nucleic acid sequences, such as those generated, for example, by using site-directed mutagenesis of SEQ ID NO: 1 or a fragment thereof. Generally, nucleic acid sequence variants of the invention will have at least 95%, at least 96%, at least 97%, at least 98% or at least 99% sequence identity to the nucleic acid sequence of SEQ ID NO: 1 or a fragment thereof as defined above, such as, for example, a nucleic acid comprising at least 700, at least 800, at least 900 or at least 950 consecutive nucleotides of the nucleotide sequence set forth as SEQ ID NO: 1. Derivatives of the nucleic acid sequences disclosed herein may include, but are not limited to, deletions of sequence, single or multiple point mutations, alterations at a particular restriction enzyme recognition site, addition of functional elements, or other means of molecular modification which may enhance, or otherwise alter promoter expression. Techniques for obtaining such derivatives are well-known in the art (see, for example, J. F. Sambrook, D. W. Russell, and N. Irwin (2000) Molecular Cloning: A Laboratory Manual). For example, one of ordinary skill in the art may delimit the functional elements within the promoters disclosed herein and delete any non-essential elements. Functional elements may be modified or combined to increase the utility or expression of the sequences disclosed herein for any particular application. Those of skill in the art are familiar with the standard resource materials that describe specific conditions and procedures for the construction, manipulation, and isolation of macromolecules (e.g., DNA molecules, plasmids, etc.), as well as the generation of recombinant organisms and the screening and isolation of DNA molecules.

The promoter sequence of SEQ ID NO: 1 and its functional fragments and variants may for example be altered to contain "enhancer DNA" to assist in elevating gene expression. As is well-known in the art, certain DNA elements can be used to enhance the transcription of DNA as part of or in association with a promoter. These enhancers are often found upstream (5') to the start of transcription in a promoter that functions in eukaryotic cells, but can be inserted downstream (3') to the coding sequence. In some instances, these enhancer DNA elements are within or part of introns. Among the introns that are useful as enhancer DNA are the 5' introns from the rice actin 1 gene (see US5641876), the rice actin 2 gene, the maize alcohol dehydrogenase gene, the maize heat shock protein 70 gene (see US5593874), the maize shrunken 1 gene, the light sensitive 1 gene of *Solanum tuberosum,* and the heat shock protein 70 gene of *Petunia hybrida* (see US 5659122). Thus, as contemplated herein, a promoter or promoter region includes variations of promoters derived by inserting or deleting regulatory regions, subjecting the promoter to random or site-directed mutagenesis etc. The activity or strength of a promoter may be measured in terms of the amounts of RNA it produces, or the amount of protein accumulation in a cell or tissue, relative to a promoter whose transcriptional activity has been previously assessed, as described above.

As used herein, the term "percent sequence identity" refers to the percentage of identical nucleotides between two segments of a window of optimally aligned DNA. Optimal alignment of sequences for aligning a comparison window are well-known to those skilled in the art and may be conducted by tools such as the local homology algorithm of Smith and Waterman (Waterman, M. S., Chapman & Hall. London, 1995), the homology alignment algorithm of Needleman and Wunsch (1970), the search for similarity method of Pearson and Lipman (1988), and preferably by computerized implementations of these algorithms such as GAP, BESTFIT, FASTA, and TFASTA available as part of the GCG (Registered Trade Mark), Wisconsin Package (Registered Trade Mark from Accelrys Inc., San Diego, Calif.). An "identity fraction" for aligned segments of a test sequence and a reference sequence is the number of identical components that are shared by the two aligned sequences divided by the total number of components in the reference sequence segment, i.e., the entire reference sequence or a smaller defined part of the reference sequence. Percent sequence identity is represented as the identity fraction times 100. The comparison of one or more DNA sequences may be to a full-length DNA sequence or a portion thereof, or to a longer DNA sequence.

The term "hybridization" refers to the ability of a first strand of nucleic acid to join with a second strand via hydrogen bond base pairing when the two nucleic acid strands have sufficient sequence identity. Hybridization occurs when the two nucleic acid molecules anneal to one another under appropriate conditions. Nucleic acid hybridization is a technique well known to those of skill in the art of DNA manipulation. The hybridization property of a given pair of nucleic acids is an indication of their similarity or identity. Another indication that two nucleic acid sequences are largely identical is that the two molecules hybridize to each other under stringent conditions. "Stringent hybridization conditions" and "stringent hybridization wash conditions" in the context of nucleic acid hybridization experiments such as Southern and Northern hybridization are sequence dependent, and are different under different environmental parameters. An example of highly stringent wash conditions is 0.1 X SSC, 5X Denhardt's solution, 0.5% SDS at 65°C for e.g. about 15 minutes. An example of appropriate wash conditions is a 2 X SSC, 0.1% SDS wash at 65°C for e.g. about 15 minutes. Often, a high stringency wash is preceded by a low stringency wash to remove background probe signal. Stringent conditions may also be achieved with the addition of destabilizing agents such as formamide. In general, a signal to noise ratio of 2 X (or higher) than that observed for an unrelated probe in the particular hybridization assay indicates detection of a specific hybridization. Very stringent conditions are selected to be equal to the Tm for a particular probe.

In the course of the present invention, a fiber-preferential promoter, together with a subsequent 5' UTR (together also denoted as "promoter region") has been identified in *Gossypium hirsutum.* Said promoter controls an Fb8-like gene in cotton.

The promoter is highly expressed in fibers. It has been shown that expression is particularly strong during the cell thickening stage of fiber development; expression of the gene product was detected from 20 to 35 dpa.

It is expected that the current promoter will be suitable for fiber-preferential expression at least during later stages of developing fibers of transgenes in cotton. The current promoter can be used to express genes which modify cotton fiber properties, especially properties of the cotton fiber cell wall.

Unless indicated otherwise, the specific definitions or specific features of certain examples disclosed in the present application in connection with one aspect can be introduced into any other aspect disclosed herein.

### Chimeric genes

In another aspect, the present application discloses a chimeric gene comprising the isolated nucleic acids described herein, i.e. the promoter and promoter regions and functional fragments, operably linked to a nucleic acid sequence coding for an expression product of interest, and a transcription termination and polyadenylation sequence functional in plant cells.

A chimeric gene is an artificial gene constructed by operably linking fragments of unrelated genes or other nucleic acid sequences. In other words "chimeric gene" denotes a gene which is not normally found in a plant species or refers to any gene in which the promoter, adjoined parts of the promoter or one or more other regulatory regions of the gene are not associated in nature with a part or all of the transcribed nucleic acid, i.e. are heterologous with respect to the transcribed nucleic acid. More particularly, a chimeric gene is an artificial, i.e. non-naturally occurring, gene produced by an operable linkage of the nucleic acid sequence of the invention, such as e.g. the nucleic acid of SEQ ID NO: 1, a fragment thereof or a nucleic acid sequence having at least 95% sequence identity thereto, all capable of directing fiber-preferential expression of an expression product of interest as described above, with a second nucleic acid sequence encoding said expression product of interest which is not naturally operably linked to said nucleic acid sequence.

The term "heterologous" refers to the relationship between two or more nucleic acid or protein sequences that are derived from different sources. For example, a promoter is heterologous with respect to an operably linked nucleic acid sequence, such as a coding sequence, if such a combination is not normally found in nature. In addition, a particular sequence may be "heterologous" with respect to a cell or organism into which it is inserted (i.e. does not naturally occur in that particular cell or organism). For example, the chimeric gene disclosed herein is a heterologous nucleic acid.

The term "operably linked" refers to the functional spatial arrangement of two or more nucleic acid regions or nucleic acid sequences. For example, a promoter region may be positioned relative to a nucleic acid sequence encoding an expression product of interest such that transcription of said nucleic acid sequence is directed by the promoter region. Thus, a promoter region is "operably linked" to the nucleic acid sequence.

The promoter, fragment or variant thereof as described above may be operably linked to a nucleic acid sequence encoding an expression product of interest that is heterologous with respect to the promoter. The nucleic acid sequence may generally be any nucleic acid sequence for which an altered level such as an increased level of transcription is desired. The nucleic acid sequence can for example encode a polypeptide that is capable of modifying fiber properties in cotton.

Suitable heterologous nucleic acid sequences for modifying the properties of cotton fibers include, without limitation, those disclosed in WO02/45485 whereby fiber quality in fiber producing plants, such as cotton, is modified by modulating sucrose synthase activity and/or expression in such plants, the nucleic acids useful for improving fiber strength or for introducing positive charges into the plant cell wall as described in WO2006/136351 or WO2011/089021.

An "expression product" denotes an intermediate or end product arising from the transcription and optionally translation of the nucleic acid, such as DNA or RNA, coding for such product. During the transcription process, a DNA sequence under control of regulatory regions, particularly the promoter sequence disclosed herein, is transcribed into an RNA molecule. An RNA molecule may either itself form an expression product and is then, for example, capable of interacting with another nucleic acid or protein. Alternatively, an RNA molecule may be an intermediate product when it is capable of being translated into a peptide or protein. A gene is said to encode an RNA molecule as expression product when the RNA as the end product of the expression of the gene is capable of interacting with another nucleic acid or protein. Examples of RNA expression products include inhibitory RNAs such as e.g. sense RNA, antisense RNA, hairpin RNA, ribozymes, miRNA or siRNA, mRNA, rRNA and tRNA. A gene is said to encode a protein or peptide as expression product when the end product of the expression of the gene is a protein or peptide.

Exemplary expression products of interest include proteins involved in cell wall synthesis and fiber formation as disclosed in WO2005/017157, in particular a gene encoding a β-1,3 glucan synthase protein, or in WO2006/136351, in particular an N-acetylglucosamine transferase which can be targeted to the membranes of the Golgi-apparatus, such as a N-acetylglucosamine transferase of the NodC type, or a chitin synthase and those described above in connection with suitable heterologous nucleic acids.

Within the scope of the present disclosure, use may also be made, in combination with the chimeric gene described above, of other regulatory sequences, which are located between said nucleic acid sequence comprising a promoter and said nucleic acid sequence comprising the coding sequence of the expression product. Non-limiting examples of such regulatory sequences include translation activators ("enhancers"), for instance the translation activator of the tobacco mosaic virus (TMV) described in Application WO 87/07644, or of the tobacco etch virus (TEV) described by Carrington & Freed 1990, J. Virol. 64: 1590-1597, or introns such as the *Arabidopsis* histone 3 intron (Chaubet *et al.,* 1992).

Other suitable regulatory sequences include 5' UTRs. As used herein, a 5'UTR, also referred to as leader sequence, is a particular region of a messenger RNA (mRNA) located between the transcription start site and the start codon of the coding region. It is involved in mRNA stability and translation efficiency. For example, the 5' untranslated leader of a petunia chlorophyll a/b binding protein gene (cab22L) downstream of the 35S transcription start site can be utilized to augment steady-state levels of reporter gene expression (Harpster et al., 1988, Mol Gen Genet. 212(1):182-90). WO95/006742 describes the use of 5' non-translated leader sequences derived from genes coding for heat shock proteins to increase transgene expression.

Such UTRs are particularly useful if the nucleic acid sequence constituting the promoter of the present invention does not comprise the first 44 or 48 nucleotides 5' of the translation start (i.e. nucleotides 926 to 959 of SEQ ID NO: 1 or nucleotides 922 to 959 of SEQ ID No 1). In other words, such UTRs may advantageously be used if the promoter of the present invention does not reach further than to nucleotide 922 or nucleotide 926 of SEQ ID NO: 1. In such cases the resulting promoter region is still capable of directing fiber-preferential or fiber-specific expression of the gene operably linked thereto in cotton between 15 and 35 days post anthesis (dpa) but not in 10 dpa fibers.

The chimeric gene also comprises a transcription termination or polyadenylation sequence operable in a plant cell, particularly a cotton plant cell. As a transcription termination or polyadenylation sequence, use may be made of any corresponding sequence of bacterial origin, such as for example the nos terminator of *Agrobacterium tumefaciens,* of viral origin, such as for example the CaMV 35S terminator, or of plant origin, such as for example a histone terminator as described in published Patent Application EP 0 633 317 A1.

In one example of the chimeric gene described herein, said expression product of interest is a protein, a peptide or an RNA molecule, said RNA molecule capable of modulating the expression of a gene endogenous to said plant. In one example, said protein, peptide or RNA molecule is capable of modulating a fiber property. In another example, said protein, peptide or RNA molecule is involved in auxin biosynthesis.

The term "protein" as used herein describes a group of molecules consisting of more than 30 amino acids, whereas the term "peptide" describes molecules consisting of up to 30 amino acids. Proteins and peptides may further form dimers, trimers and higher oligomers, i.e. consisting of more than one (poly)peptide molecule. Protein or peptide molecules forming such dimers, trimers etc. may be identical or non-identical. The corresponding higher order structures are, consequently, termed homo- or heterodimers, homo- or heterotrimers etc. The terms "protein" and "peptide" also refer to naturally modified proteins or peptides wherein the modification is effected e.g. by glycosylation, acetylation, phosphorylation and the like. Such modifications are well known in the art.

Example proteins suitable as expression products include proteins involved in the modification of fiber properties, such as those mediating an increase in fiber length, an alteration in fiber strength or an alteration in the cell wall properties resulting, e.g. in an altered charge of said cell walls, an alteration in dyeability, decreased fuzz fiber production, an alteration in the fiber maturity ratio, a decrease in immature fiber content, or an increase in fiber uniformity and micronaire.

Said expression product of interest may also be an RNA molecule capable of modulating the expression of a gene endogenous to said cotton plant.

Examples of target genes suitable for RNA expression products in this connection include those involved in the modification of fiber properties, such as those mediating an increase in fiber length, an alteration in fiber strength or an alteration in the cell wall properties resulting, e.g. in an altered charge of said cell walls, an alteration in dyeability, decreased fuzz fiber production, an alteration in the fiber maturity ratio, a decrease in immature fiber content, or an increase in fiber uniformity and micronaire.

For the case of RNA molecules, it will be clear that whenever nucleic acid sequences of RNA molecules are defined by reference to nucleic acid sequences of corresponding DNA molecules, the thymine (T) in the nucleic acid sequence should be replaced by uracil (U). Whether reference is made to RNA or DNA molecules will be clear from the context of the application.

The term "capable of modulating the expression of a gene" relates to the action of an RNA molecule, such as an inhibitory RNA molecule as described herein, to influence the expression level of target genes in different ways. This can be effected e.g. by inhibiting the expression of a target gene by directly interacting with components driving said expression such as the gene itself or the transcribed mRNA which results in a decrease of expression, or by inhibiting another gene involved in activating the expression of a target gene thereby abolishing said activation, or inhibiting another gene involved in inhibiting the expression of a target gene which results in an increase of expression. The inhibition of a gene involved in inhibiting the expression of a target gene using inhibitory RNA may, on the contrary, result in an activation of expression of said target gene.

Inhibitory RNA molecules decrease the levels of mRNAs of their target proteins available for translation into said target protein. In this way, expression of proteins involved in unwanted responses to stress conditions can be inhibited. This can be achieved through well-established techniques including co-suppression (sense RNA suppression), antisense RNA, double-stranded RNA (dsRNA), siRNA or microRNA (miRNA).

An RNA molecule as expression product as disclosed herein comprises a part of a nucleic acid sequence encoding a target protein or a homologous sequence to down-regulate the expression of said target protein. Another example for an RNA molecule as expression product for use in down-regulating expression are antisense RNA molecules comprising a nucleic acid sequence complementary to at least a part of a nucleic acid sequence encoding a protein of interest or a homologous sequence. Here, down-regulation may be effected e.g. by introducing this antisense RNA or a chimeric DNA encoding such RNA molecule. In yet another example, expression of a protein of interest is down-regulated by introducing a double-stranded RNA molecule comprising a sense and an antisense RNA region corresponding to and respectively complementary to at least part of a gene sequence encoding said protein of interest, which sense and antisense RNA region are capable of forming a double stranded RNA region with each other. Such double-stranded RNA molecule may be encoded both by sense and antisense molecules as described above and by a single-stranded molecule being processed to form siRNA or miRNA.

In one example, expression of a target protein may be down-regulated by introducing a chimeric DNA construct which yields a sense RNA molecule capable of down-regulating expression by co-suppression. The transcribed DNA region will yield upon transcription a so-called sense RNA molecule capable of reducing the expression of a gene encoding a target protein in the target plant or plant cell in a transcriptional or post-transcriptional manner. The transcribed DNA region (and resulting RNA molecule) comprises at least 20 consecutive nucleotides having at least 95% sequence identity to the corresponding portion of the nucleic acid sequence encoding the target protein present in the plant cell or plant.

Alternatively, an expression product for down-regulating expression of a target protein is an antisense RNA molecule. Down-regulating or reducing the expression of a protein of interest in the target cotton plant or plant cell is again effected in a transcriptional or post-transcriptional manner. The transcribed DNA region (and resulting RNA molecule) comprises at least 20 consecutive nucleotides having at least 95% sequence identity to the complement of the corresponding portion of the nucleic acid sequence encoding said target protein present in the plant cell or plant.

However, the minimum nucleic acid sequence of the antisense or sense RNA region of about 20 nt (nucleotides) of the nucleic acid sequence encoding a target protein may be comprised within a larger RNA molecule, varying in size from 20 nt to a length equal to the size of the target gene. The mentioned antisense or sense nucleic acid regions may thus be from about 21 nt to about 5000 nt long, such as 21 nt, 40 nt, 50 nt, 100 nt, 200 nt, 300 nt, 500 nt, 1000 nt, 2000 nt or even about 5000 nt or larger in length. Moreover, it is not required for the purpose of the invention that the nucleic acid sequence of the used inhibitory RNA molecule or the encoding region of the transgene, is completely identical or complementary to the endogenous gene encoding the target protein the expression of which is targeted to be reduced in the plant cell. The longer the sequence, the less stringent the requirement for the overall sequence identity is. Thus, the sense or antisense regions may have an overall sequence identity of about 40 % or 50 % or 60 % or 70 % or 80 % or 90 % or 100 % to the nucleic acid sequence of an endogenous gene or the complement thereof. However, as mentioned, antisense or sense regions should comprise a nucleic acid sequence of 20 consecutive nucleotides having about 95 to about 100 % sequence identity to the nucleic acid sequence of the endogenous gene encoding the target gene. The stretch of about 95 to about 100% sequence identity may be about 50, 75 or 100 nt.

The efficiency of the above mentioned chimeric genes for antisense RNA or sense RNA-mediated gene expression level down-regulation may be further enhanced by inclusion of DNA elements which result in the expression of aberrant, non-polyadenylated inhibitory RNA molecules. One such DNA element suitable for that purpose is a DNA region encoding a self-splicing ribozyme. The efficiency may also be enhanced by providing the generated RNA molecules with nuclear localization or retention signals.

In addition, an expression product as described herein may be a nucleic acid sequence which yields a double-stranded RNA molecule capable of down-regulating expression of a gene encoding a target protein. Upon transcription of the DNA region the RNA is able to form dsRNA molecule through conventional base paring between a sense and antisense region, whereby the sense and antisense region are nucleic acid sequences as hereinbefore described. Expression products being dsRNA according to the invention may further comprise an intron, such as a heterologous intron, located e.g. in the spacer sequence between the sense and antisense RNA regions in accordance with the disclosure of WO 99/53050. To achieve the construction of such a transgene, use can be made of the vectors described in WO 02/059294 A1.

In an example, said RNA molecule comprises a first and second RNA region wherein 1. said first RNA region comprises a nucleic acid sequence of at least 19 consecutive nucleotides having at least about 94% sequence identity to the nucleic acid sequence of said endogenous gene; 2. said second RNA region comprises a nucleic acid sequence complementary to said 19 consecutive nucleotides of said first RNA region; 3. said first and second RNA region are capable of base-pairing to form a double stranded RNA molecule between at least said 19 consecutive nucleotides of said first and second region. In other examples, the same considerations apply as described above for sense and antisense RNA.

Another example expression product is a microRNA molecule (miRNA, which may be processed from a pre-microRNA molecule) capable of guiding the cleavage of mRNA transcribed from the DNA encoding the target protein which is to be translated into said target protein. miRNA molecules may be conveniently introduced into plant cells through expression from a chimeric gene as described herein comprising a (second) nucleic acid sequence encoding as expression product of interest such miRNA, pre-miRNA or primary miRNA transcript.

miRNAs are small endogenous RNAs that regulate gene expression in plants, but also in other eukaryotes. As used herein, a "miRNA" is an RNA molecule of about 20 to 30 nucleotides (Siomi and Siomi, 2009) in length which can be loaded into a RISC complex and direct the cleavage of a target RNA molecule, wherein the target RNA molecule comprises a nucleic acid sequence essentially complementary to the nucleic acid sequence of the miRNA molecule. In example miRNAs, one or more of the following mismatches in the miRNA essentially complementary to the target RNA may occur:
- A mismatch between the nucleotide at the 5' end of said miRNA and the corresponding nucleic acid sequence in the target RNA molecule;
- A mismatch between any one of the nucleotides in position 1 to position 9 of said miRNA and the corresponding nucleic acid sequence in the target RNA molecule;
- Three mismatches between any one of the nucleotides in position 12 to position 21 of said miRNA and the corresponding nucleic acid sequence in the target RNA molecule provided that there are no more than two consecutive mismatches;
- No mismatch is allowed at positions 10 and 11 of the miRNA (all miRNA positions are indicated starting from the 5' end of the miRNA molecule).

As used herein, a "pre-miRNA" molecule is an RNA molecule of about 100 to about 200 nucleotides, preferably about 100 to about 130 nucleotides which can adopt a secondary structure comprising a dsRNA stem and a single stranded RNA loop and further comprising the nucleic acid sequence of the miRNA and its complement sequence of the miRNA* in the double-stranded RNA stem. Preferably, the miRNA and its complement are located about 10 to about 20 nucleotides from the free ends of the miRNA dsRNA stem. The length and sequence of the single stranded loop region are not critical and may vary considerably, e.g. between 30 and 50 nt in length. Preferably, the difference in free energy between unpaired and paired RNA structure is between -20 and -60 kcal/mole, for example around -40 kcal/mole. The complementarity between the miRNA and the miRNA* does not need to be perfect and about 1 to 3 bulges of unpaired nucleotides can be tolerated. The secondary structure adopted by an RNA molecule can be predicted by computer algorithms conventional in the art such as mFold, UNAFold and RNAFold. The particular strand of the dsRNA stem from the pre-miRNA which is released by DCL activity and loaded onto the RISC complex is determined by the degree of complementarity at the 5' end, whereby the strand which at its 5' end is the least involved in hydrogen bonding between the nucleotides of the different strands of the cleaved dsRNA stem is loaded onto the RISC complex and will determine the sequence specificity of the target RNA molecule degradation. However, if empirically the miRNA molecule from a particular synthetic pre-miRNA molecule is not functional because the "wrong" strand is loaded on the RISC complex, it will be immediately evident that this problem can be solved by exchanging the position of the miRNA molecule and its complement on the respective strands of the dsRNA stem of the pre-miRNA molecule. As is known in the art, binding between A and U involving two hydrogen bounds, or G and U involving two hydrogen bounds is less strong that between G and C involving three hydrogen bounds.

miRNA molecules may be comprised within their naturally occurring pre-miRNA molecules but they can also be introduced into existing pre-miRNA molecule scaffolds by exchanging the nucleic acid sequence of the miRNA molecule normally processed from such existing pre-miRNA molecule for the nucleic acid sequence of another miRNA of interest. The scaffold of the pre-miRNA can also be completely synthetic. Likewise, synthetic miRNA molecules may be comprised within, and processed from, existing pre-miRNA molecule scaffolds or synthetic pre-miRNA scaffolds.

Example expression products can also be ribozymes catalyzing either their own cleavage or the cleavage of other RNAs.

In one example of the chimeric gene disclosed herein modulating the expression is increasing the expression and said nucleic acid sequence encoding an expression product of interest encodes an RNA, which when transcribed yields an RNA molecule capable of increasing the expression of a gene endogenous to said cotton plant. Such genes could be positively correlated with fiber length, fiber strength or a desired alteration in the cell wall properties resulting, e.g. in an altered charge of said cell walls, an alteration in dyeability, decreased fuzz fiber production, an alteration in the fiber maturity ratio, a decrease in immature fiber content, or an increase in fiber uniformity and micronaire, or 2. yields an RNA molecule capable of decreasing the expression of a gene endogenous to said cotton plant, wherein said gene may be negatively correlated with fiber length, fiber strength or a desired alteration in the cell wall properties resulting, e.g. in an altered charge of said cell walls, an alteration in dyeability, decreased fuzz fiber production, an alteration in the fiber maturity ratio, a decrease in immature fiber content, or an increase in fiber uniformity and micronaire.

In another example of the chimeric gene described herein, said expression product is a reporter gene or a fiber-specific gene as described elsewhere in this application.

In one example of the chimeric gene described herein, said RNA molecule comprises a first and second RNA region wherein 1. said first RNA region comprises a nucleic acid sequence of at least 19 consecutive nucleotides having at least about 94% sequence identity to the nucleic acid sequence of said endogenous gene; 2. said second RNA region comprises a nucleic acid sequence complementary to said 19 consecutive nucleotides of said first RNA region; and 3. said first and second RNA region are capable of base-pairing to form a double stranded RNA molecule between at least said 19 consecutive nucleotides of said first and second region.

### Plant cells and plants

The present invention is also directed to transgenic cotton plant cells and transgenic cotton plants which comprise a nucleic acid sequence as described above, i.e. the promoter sequence disclosed herein, operably linked to a heterologous nucleic acid sequence encoding an expression product of interest. Alternatively, said transgenic plant cells or plants comprise the chimeric gene disclosed herein. Example promoter sequences and expression products of interest and other regulatory elements, are described above.

A transgenic cotton plant cell or cotton plant may be produced by introducing the nucleic acid sequence(s) as described above into cotton plants or cotton plant cells. "Introducing" in connection with the present application relates to the placing of genetic information in a plant cell or plant by artificial means. This can be effected by any method known in the art for introducing RNA or DNA into plant cells, protoplasts, calli, roots, tubers, seeds, stems, leaves, seedlings, embryos, pollen and microspores, other plant tissues, or whole plants. More particularly, "introducing" means stably integrating into the plant's genome.

Plants containing at least one transformed nucleic acid sequence are referred to as "transgenic plants". Transgenic and recombinant refer to a host organism such as a plant into which a heterologous nucleic acid molecule (e.g. the nucleic acid sequence, the chimeric gene as described herein) has been introduced. The nucleic acid can be stably integrated into the genome of the plant. Specific methods for introduction are described in connection with the methods disclosed herein.

"Cotton" or "cotton plant" as used herein can be any species from the genus *Gossypium* useful for growing harvesting cotton fibers. The most commonly used cotton species are *Gossypium barbadense, G. hirsutum, G. arboreum* and *G. herbaceum.* Further species include *G. africanum* and *G. raimondii.* Also included are progeny from crosses of any of the above species with other species or crosses between such species.

A cotton plant cell may be any cell comprising essentially the genetic information necessary to define a cotton plant, which may, apart from the chimeric gene disclosed herein, be supplemented by one or more further transgenes. Cells may be derived from the various organs and/or tissues forming a cotton plant, including but not limited to fruits, seeds, embryos, reproductive tissue, meristematic regions, callus tissue, leaves, roots, shoots, flowers, vascular tissue, gametophytes, sporophytes, pollen, and microspores.

The present application also discloses a transgenic plant consisting of the transgenic cotton plant cells described hereinabove, or comprising the chimeric gene described herein stably integrated in the plant genome. This may be effected by transformation protocols described elsewhere in this application.

In another embodiment, the present invention relates to a seed generated from a transgenic plant described herein, wherein said seed comprises the chimeric gene described herein.

Seed is formed by an embryonic plant enclosed together with stored nutrients by a seed coat. It is the product of the ripened ovule of gymnosperm and angiosperm plants, to the latter of which cotton belongs, which occurs after fertilization and to a certain extent growth within the mother plant.

Further disclosed herein are cotton fibers and cotton seed oil obtainable or obtained from the plants disclosed herein. Cotton fibers disclosed herein can be distinguished from other fibers by applying the detection method disclosed in WO2010/015423 and checking for the presence of the nucleic acid of (a) or chimeric gene of (b) in the fibers. Accordingly, the nucleic acid of (a) may also be used for tracking cell walls, in particular cotton fibers according to the invention.

### Methods and uses

The present invention furthermore relates to a method of producing a transgenic cotton plant (a) providing a chimeric gene described herein; and (b) introducing said chimeric gene in a plant.

A number of methods are available to introduce DNA into plant cells or plants, either by transformation or introgression. Agrobacterium-mediated transformation of cotton has been described e.g. in US patent 5,004,863, in US patent 6,483,013 and WO 2000/71733.

Plants may also be transformed by particle bombardment: Particles of gold or tungsten are coated with DNA and then shot into young plant cells or plant embryos. This method also allows transformation of plant plastids. Cotton transformation by particle bombardment is reported e.g. in WO 92/15675.

Viral transformation (transduction) may be used for transient or stable expression of a gene, depending on the nature of the virus genome. The desired genetic material is packaged into a suitable plant virus and the modified virus is allowed to infect the plant. The progeny of the infected plants is virus free and also free of the inserted gene. Suitable methods for viral transformation are described or further detailed e.g. in WO 90/12107, WO 03/052108 or WO 2005/098004.

"Introgressing" means the integration of a gene in a plant's genome by natural means, i.e. by crossing a plant comprising the chimeric gene described herein with a plant not comprising said chimeric gene. The offspring can be selected for those comprising the chimeric gene.

Further transformation and introgression protocols can also be found in US patent 7,172,881.

The chimeric gene may be introduced, e.g. by transformation, in cotton plants from which embryogenic callus can be derived, such as Coker 312, Coker310, GSC25110, FIBERMAX 819, Siokra 1-3, T25, GSA75, Acala SJ2, Acala SJ4, Acala SJ5, Acala SJ-C1, Acala B1644, Acala B1654-26, Acala B1654-43, Acala B3991, Acala GC356, Acala GC510, Acala GAM1, Acala C1, Acala Royale, Acala Maxxa, Acala Prema, Acala B638, Acala B1810, Acala B2724, Acala B4894, Acala B5002, non Acala "picker" Siokra, "stripper" variety FC2017, Coker 315, STONEVILLE 506, STONEVILLE 825, DP50, DP61, DP90, DP77, DES119, McN235, HBX87, HBX191, HBX107, FC 3027, CHEMBRED A1, CHEMBRED A2, CHEMBRED A3, CHEMBRED A4, CHEMBRED B1, CHEMBRED B2, CHEMBRED B3, CHEMBRED C1, CHEMBRED C2, CHEMBRED C3, CHEMBRED C4, PAYMASTER 145, HS26, HS46, SICALA, PIMA S6 ORO BLANCO PIMA, FIBERMAX FM5013, FIBERMAX FM5015, FIBERMAX FM5017, FIBERMAX FM989, FIBERMAX FM832, FIBERMAX FM966, FIBERMAX FM958, FIBERMAX FM989, FIBERMAX FM958, FIBERMAX FM832, FIBERMAX FM991, FIBERMAX FM819, FIBERMAX FM800, FIBERMAX FM960, FIBERMAX FM966, FIBERMAX FM981, FIBERMAX FM5035, FIBERMAX FM5044, FIBERMAX FM5045, FIBERMAX FM5013, FIBERMAX FM5015, FIBERMAX FM5017 or FIBERMAX FM5024 and plants with genotypes derived thereof.

In a further aspect, the present application discloses a method of growing cotton comprising (a1) providing the transgenic plant described herein or produced by the method described herein; or (a2) introducing a chimeric gene described herein in a plant; (b) growing the plant of (a1) or (a2); and (c) harvesting cotton produced by said plant.

"Growing" relates to creating the environment for plants to grow, multiply and/or age. Suitable growing conditions for specific plants are well-known in the art.

In another aspect, the present application discloses to a method of producing a seed comprising the chimeric gene disclosed herein comprising (a) growing a transgenic plant comprising the chimeric gene described herein or the vector described herein, a transgenic plant described herein or a transgenic plant obtained by the method described herein, wherein said transgenic plant produces said seed and said chimeric gene is comprised in said seed, and (b) isolating said seed from said transgenic plant.

In one example of the method of producing a transgenic plant or the method of producing a seed, the plant is a cotton plant as described elsewhere in this application.

In another aspect, the present application discloses to a method of effecting fiber-preferential expression of a product in cotton between 15 and 35 days post anthesis (dpa) but not in 10 dpa fibers comprising introducing the chimeric gene disclosed herein into the genome of a cotton plant; or providing the transgenic cotton plant disclosed herein.

In a further aspect, the present application discloses a method of altering fiber properties in a cotton plant comprising introducing the chimeric gene disclosed herein into the genome of a cotton plant; or providing the transgenic cotton plant disclosed herein.

In one example, the method further comprises growing said plant until seed are generated.

In another example based on the above further step, the method is for increasing cotton yield from a cotton plant and further comprises harvesting the cotton produced by said cotton plant. In other words disclosed herein is a method for increasing cotton yield from a cotton plant comprising introducing the chimeric gene disclosed herein into the genome of a cotton plant; or providing the transgenic plant disclosed herein; growing said plant until seed are generated; and harvesting the cotton produced by said cotton plant.

The term "increasing the yield" in connection with the present application relates to an increase in the output of cotton fibers which can be achieved e.g. by increasing the number of fibers produced on a cotton seed, the length of the fibers or the strength of the fibers. Genes and expression products thereof involved in conferring these properties have been described above.

Example features for a modification of fiber properties are fiber length, fiber strength, an alteration in the cell wall properties resulting, e.g. in an altered charge of cell walls, dyeability, decreased fuzz fiber production or content, fiber maturity ratio, immature fiber content, fiber uniformity and micronaire.

In another aspect, the present application discloses the use of the chimeric gene disclosed herein or the transgenic cotton plant or plant cell disclosed herein for fiber-preferential expression of a product in cotton between 15 and 35 days post anthesis (dpa) but not in 10 dpa fibers, for altering fiber properties in cotton or for increasing cotton yield. The definitions and further examples described above for other aspects disclosed herein equally apply to the present aspect.

The transformed plant cells and plants described herein such as those obtained by the methods described herein may be further used in breeding procedures well known in the art, such as crossing, selfing, and backcrossing. Breeding programs may involve crossing to generate an F1 (first filial) generation, followed by several generations of selfing (generating F2, F3, etc.). The breeding program may also involve backcrossing (BC) steps, whereby the offspring is backcrossed to one of the parental lines, termed the recurrent parent.

Accordingly, also disclosed herein is a method for producing plants comprising the chimeric gene disclosed herein comprising the step of crossing the cotton plant disclosed herein with another plant or with itself and selecting for offspring comprising said chimeric gene.

The transgenic plant cells and plants obtained by the methods disclosed herein may also be further used in subsequent transformation procedures, e.g. to introduce a further chimeric gene.

The transgenic plant cells and plants comprising a recombinant gene according to the invention may be used to produce food, feed (such as oil, meal, grain, starch, flour or protein) or an industrial product such as biofuel, fiber, industrial chemicals, a pharmaceutical or a nutraceutical, particularly for the production of cotton fibers.

The cotton plants or seed comprising the chimeric gene disclosed herein or obtained by the methods disclosed herein may further be treated with cotton herbicides such as Diuron, Fluometuron, MSMA, Oxyfluorfen, Prometryn, Trifluralin, Carfentrazone, Clethodim, Fluazifop-butyl, Glyphosate, Norflurazon, Pendimethalin, Pyrithiobac-sodium, Trifloxysulfuron, Tepraloxydim, Glufosinate, Flumioxazin, Thidiazuron; cotton insecticides such as Acephate, Aldicarb, Chlorpyrifos, Cypermethrin, Deltamethrin, Abamectin, Acetamiprid, Emamectin Benzoate, Imidacloprid, Indoxacarb, Lambda-Cyhalothrin, Spinosad, Thiodicarb, Gamma-Cyhalothrin, Spiromesifen, Pyridalyl, Flonicamid, Flubendiamide, Triflumuron, Rynaxypyr, Beta-Cyfluthrin, Spirotetramat, Clothianidin, Thiamethoxam, Thiacloprid, Dinetofuran, Flubendiamide, Cyazypyr, Spinosad, Spinotoram, gamma Cyhalothrin, 4-[[(6-Chlorpyridin-3-yl)methyl](2,2-difluorethyl)amino]furan-2(5H)-on, Thiodicarb, Avermectin, Flonicamid, Pyridalyl, Spiromesifen, Sulfoxaflor; and cotton fungicides such as Azoxystrobin, Bixafen, Boscalid, Carbendazim, Chlorothalonil, Copper, Cyproconazole, Difenoconazole, Dimoxystrobin, Epoxiconazole, Fenamidone, Fluazinam, Fluopyram, Fluoxastrobin, Fluxapyroxad, Iprodione, Isopyrazam, Isotianil, Mancozeb, Maneb, Metominostrobin, Penthiopyrad, Picoxystrobin, Propineb, Prothioconazole, Pyraclostrobin, Quintozene, Tebuconazole, Tetraconazole, Thiophanate-methyl, Trifloxystrobin. For a treatment with cotton herbicides, said cotton plants or seed preferably further comprise a trait conferring a respective herbicide tolerance or are naturally tolerant to a herbicide.

The following non-limiting Examples describe the isolation of a fiber-selective promoter, and the construction of chimeric genes for selective expression in developing fiber cells. Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Croy, jointly published by BIOS Scientific Publications Ltd (UK) and Blackwell Scientific Publications, UK.

Throughout the description and Examples, reference is made to the following sequences represented in the sequence listing:
SEQ ID No 1: nucleotide sequence of the fiber selective promoter region of an FB8-like gene from Gossypium hirsutum (BAC8K12)
SEQ ID No. 2: nucleotide sequence of oligonucleotide primer FB8-like_F
SEQ ID No. 3: nucleotide sequence of oligonucleotide primer FB8-like_R
SEQ ID No. 4: nucleotide sequence of oligonucleotide primer QFB8-like_F
SEQ ID No. 5: nucleotide sequence of oligonucleotide primer QFB8-like_R
SEQ ID No. 6: nucleotide sequence of oligonucleotide primer UbiQ_F
SEQ ID No. 7: nucleotide sequence of oligonucleotide primer UbiQ_R
SEQ ID No. 8: nucleotide sequence of oligonucleotide primer gwFb8-likePro(8K12)_F
SEQ ID No. 9: nucleotide sequence of oligonucleotide primer gwFb8-likePro(8K12)_R
SEQ ID No. 10: nucleotide sequence of the FB8-like 8K12 gene (promoter region and coding region)
SEQ ID No. 11: nucleic acid sequence encoding the Neurospora crassa chitin synthase 2 gene
SEQ ID No. 12: Golgi-targeting signal from Arabidopsis thaliana
SEQ ID No. 13: coding sequence of the gfa (glutamine:fructose-6-phosphate amidotransferase) gene from E. coli
SEQ ID No. 14: nucleotide sequence of pTIB259

### Examples

### Example 1: Identification of candidate genes expressed preferentially in fibers

A list of about 450 potential fiber expressed genes was generated from an analysis of the literature and public microarray data and this was narrowed down through a process of examining the frequency of occurrence of ESTs homologous to those genes in public databases (as a surrogate of transcript abundance) and then followed up with quantitative RT-PCR across a range of tissue types and fiber ages. It was decided to particularly look at the gene F203F9 (DR176749, Fb8-like). This is a gene originally identified by Liu et al. (2006) by suppression-subtractive hybridisation from the fibers of *G. hirsutum* cultivar TM-1. Liu et al., 2006 noted that clone F203F9 (lodged as Genbank accession DR176749) was predominantly expressed in 20 dpa fibers, but not expressed in 10 dpa fibers. In that publication the EST was annotated as very similar to the *G. barbadense* cDNA Fb8-like (AF531368), but is also closely related to the *G. barbadense* FbLate-2 (U34401) cDNA, both members of a multigene family in cotton.

A blastN search of the Genbank cotton ESTs with DR176749 identified several very similar ESTs, including AF531368, confirming that this gene was a member of a multigene family. DR176749 was identical over most of its length to the longer EST sequence of AF531368 so this was used to design primers (Fb8-like_F: 5'-attcttttctttctatttggttaaccat-3' (SEQ ID NO: 2) and Fb8-like_R: 5'-tagtgctcgagccagactga-3' (SEQ ID NO: 3)) that flanked the coding region of AF531368 Fb8-like EST to amplify the gene(s) from Upland cotton fiber cDNA and genomic DNA to examine the diversity within this gene family.

Both cDNA (from a mix of 10 and 20 dpa Coker315 fiber mRNA) and genomic FB8-like clones (from Coker315 DNA) were cloned and sequenced and these varied in both length and sequence, confirming the multigenic nature of this family of Fb8-like genes. Three similar but different length clones, for example, were recovered from fiber cDNA with clone cFB8-like #1 being the closest in identity to AF531368, the *G. barbadense* FB8-like gene.

This cDNA encoded a predicted protein of 276 amino acids considerably longer than the 165 amino acid residues of the *G. barbadense* Fb8-like protein but very similar in sequence.

The protein has no recognized conserved domains (as confirmed by using the prediction platform provided at http://www.ncbi.nlm.nih.gov/Structure/cdd/wrpsb.cgi), but has a predicted signal peptide for secretion (as confirmed using the prediction platform provided at http://www.cbs.dtu.dk/services/SignalP/) and has an interesting repeat sequence structure of three glutamate-lysine rich domains as predicted by the RADAR website (http://www.ebi.ac.uk/Tools/Radar/). The *G. barbadense* FB8-like protein has only the first of these three repeats.

Q-PCR primers were designed to near the C-terminal end of a consensus FB8-like sequence and used to profile the relative expression of Fb8-like genes in different tissues of the *G. hirsutum* cultivar Coker 315 using the ubiquitin gene as a reference. RNA preparation was carried out according to Wan and Wilkins (1994) with up to 2 g ground fibers or other tissues. All RNA preparations were DNAse treated and purified by organic extraction. Real time PCR experiments used 5 µg of total RNA for the production of cDNA according to the manufacturer's recommendations (Superscript II, BRL Life Technologies, Gaithersburg, MD, USA) and were primed with 0.5µg of oligo-dT (dT18).

The primers used were designed with Primer 3 (http://frodo.wi.mit.edu/cgi-bin/primer3/primer3_www.cgi) and were QFb8like_F: 5'-gagtgccaagagtcacacga-3' (SEQ ID NO: 4) and QFb8like_R: 5'-ttggatcccacccttaaaca-3' (SEQ ID NO: 5) that amplify a product of 113 bp. The primers for the cotton Ubiquitin gene were UbiQ_F: 5'-aatccactttgcacctggtc -3' (SEQ ID NO: 6) and UbiQ_R: 5'- ctagtggccagctcacatca -3' (SEQ ID NO: 7) that amplify a product of 131 bp (Al Ghazi et al., 2009). Quantitative Real-time PCR experiments were carried out in an Applied Biosystems 7900HT Fast Real-time PCR system (CA.USA) according to the following procedure. 15 µL of a master mix consisting of 10µl of 2X SYBR Green JumpStart Taq Ready Mix (Sigma), 0.5µL of each 20µM forward and reverse oligonucleotides corresponding to the given target gene and 4 µL of PCR grade water were pipetted into 96 well plates. The templates (5µL of a 1/500 dilution of the cDNA reaction) were then added to the master mixes and transferred to the thermal cycler. Cycling conditions were 5 min of denaturation at 95°C followed by 40 cycles of 95°C denaturation for 15 seconds, 60°C annealing for 15 seconds and 72°C elongation for 20seconds. Following amplification, a dissociation stage was carried out to detect any complex products. Data analysis was performed with RQmanager V1.2 software (Applied Biosystems) and relative transcript abundance compared to 35 dpa fibers determined using the cotton ubiquitin gene as a reference gene to correct for cDNA input using the ΔΔCt method.

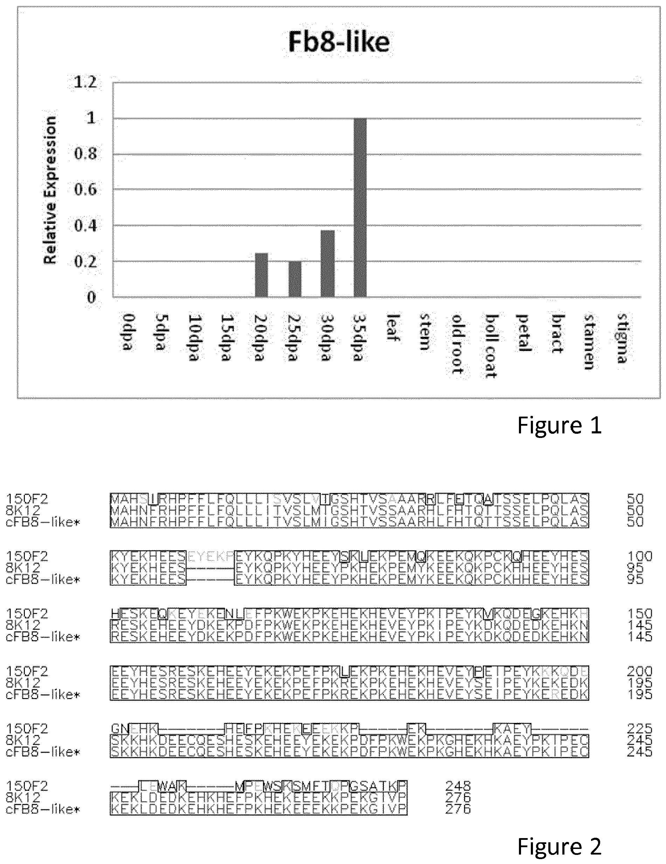

According to the Q-PCR data (Figure 1), the Fb8-Like family is most highly expressed in the cell wall thickening stage of fiber development (from 20 through to 35 dpa), although highest in about 35 dpa fibers, with little or no expression elsewhere.

### Example 2: Isolation of an Fb8-like promoter

The insert from the cFB8-like#1 cDNA clone was excised, gel purified and used as a probe to screen a large insert BAC library from *G. hirsutum* cv Acala Maxxa (GH_MBb, Clemson Genomics Institute) according to the suppliers recommended protocol (http://www.genome.clemson.edu/resources/protocols). Several strong hybridising BACs were obtained, DNA prepared and grouped by restriction pattern type using Southern blotting. Sequencing with primers in the coding region of the clones with unique restriction patterns identified the clone BAC8K12. BAC8K12 encoded a protein almost identical to cFb8-like#1 (Figure 2).

Sequencing upstream into the promoter and down into the coding region was carried out in a stepwise manner from BAC DNA. The encoded gene did not appear to contain an intron and encoded a protein of 277 amino acids. The sequence of the promoter and gene for BAC8K12 is shown in SEQ ID NO: 10 (coding region in capitals; the promoter region used for the reporter expression construct is underlined. Regions contained within the gateway primers used to amplify the promoter from BAC DNA are indicated in bold):

### Example 3: Production of an expression construct comprising pFb8-like operably linked to GUS

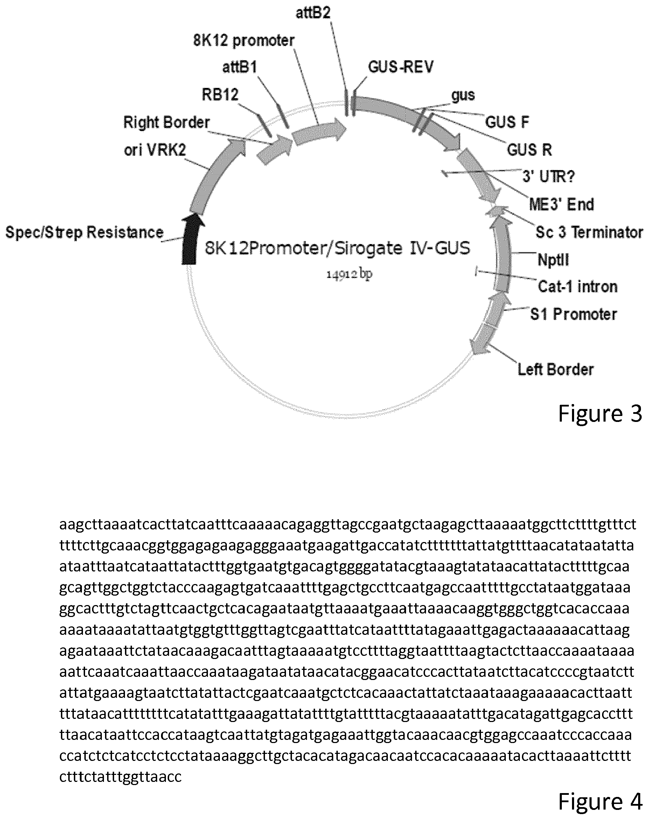

A 959 bp fragment of the promoter region upstream from the ATG start codon was amplified from BAC DNA of BAC8K12 using the gateway primers gwFb8-likePro(8K12)_F (SEQ ID No. 8):
5'-ggggacaagtttgtacaaaaaagcaggct**aagcttaaaatcacttatcaatttcaaaaacaga** - 3' and gwFb8-likePro(8K12)_R (SEQ ID No. 9):
5'-ggggaccactttgtacaagaaagct**gggtggttaaccaaatagaaagaaaagaattttaagt**-3' (gene-specific sequences are in bold) containing the attB1 and attB2 recombination sites for *in vitro* recombination using the Gateway cloning system (Invitrogen). PCR amplification from BAC DNA followed by PEG cleanup was according to the manufacturer's instructions and *in vitro* recombination using the BP reaction with the intermediate vector pDONR201 (Invitrogen) and transformation into DH5alpha cells resulted in the production of pDONR/ gwFb8-like(8K12). The intermediate vector was sequenced with pDONR-F and pDONR-R primers (Invitrogen) and then recombined into a GUS reporter gene destination vector using the LR reaction as recommended. This vector contains the left and right borders of the T-DNA, a promoterless GUS gene downstream of an attR gateway recombination cassette, a terminator from the *Flavaria bidentis* malic enzyme and a plant expressible kanamycin resistance gene with a promoter from the segment 1 sub-genomic RNA and terminator of segment 3 of the sub-clover stunt virus as described in Schünmann et al., (2003). The resultant plasmid pFb8-like(812K)- GUS (Figure 3) was sequenced with a primer in the GUS reporter gene to confirm the correct recombination of the promoter and then transferred to *Agrobacterium* strain AGL1 (Lazo et al., 1991) by electroporation.

The sequence of the promoter in 5'-3' direction (Fig 4) corresponds to SEQ ID NO: 1

### Example 4: Expression of a construct comprising pFb8-like promoter region operably linked to GUS

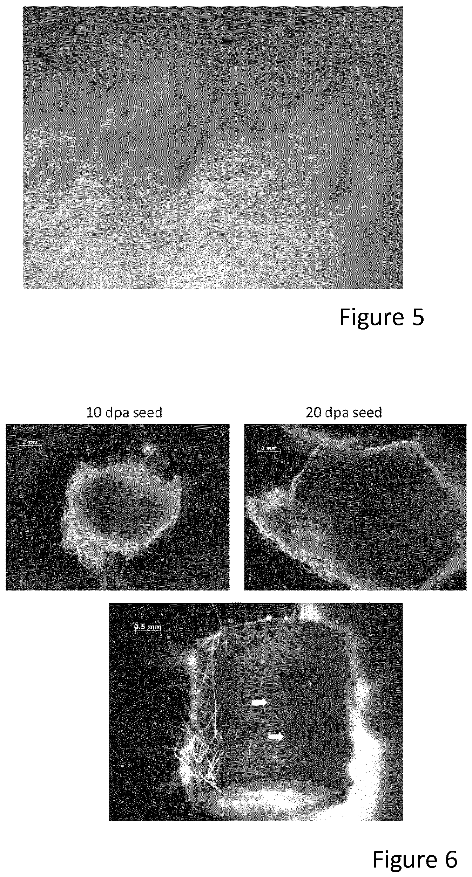

Functionality of the promoter was tested by bombardment of pFb8-like(8K12)-GUS vector into cultured cotton ovules and staining for GUS enzyme activity (Jefferson et al., 1987). Gus activity was assayed at 7dpa, 21dpa and 28dpa fibers (see Table 1 and Figure 5).

**Table 1: number of GUS spots/number of ovules analyzed. The Fb8-like(8K12) promoter was able to drive GUS expression between 16dpa and 29dpa.**

| **Promoter** | 7dpa | 16dpa | 29dpa |
|---|---|---|---|
| 35S | 40/10 | 1/18 | not tested |
| Fb8-like(8K12) | Not tested | 1/40 | 5/48 |

Cotton transformation with the *Agrobacterium* strain carrying the pFb8-like(8K12) -GUS vector was carried out essentially as described in Murray et al., (1999) using the seedling cotyledon segments of cotton cultivar Coker315-11 (a selection from the regenerable Coker 315 cultivar) and selection for resistance to kanamycin sulphate.

A total of 22 primary transformants were generated representing 11 independent transformation events. Plants were transferred to soil and allowed to flower. Flowers were tagged at anthesis and both 10 and 20 dpa bolls collected and whole seeds with fibers stained for GUS enzyme using X-gluc histochemical stains as described by Jefferson et al., 1987. Immature leaves, petiole segments, roots and stem sections from both young and older branches were also stained for GUS. Staining patterns for four different independent lines are summarized in Table 2 and representative images are provided in Figure 6.

**Table 2: Tissues in which GUS enzyme activity were detected in four different T₀ transgenic cotton plants transformed with the pFb8-like(8K12)-GUS construct. +, weak or localised expression, ++ medium expression, +++ very strong expression.**

| Transformant line | Leaf | Petiole | Stem | Root | 10dpa fiber | 20dpa fiber |
|---|---|---|---|---|---|---|
| T447-1-1 | - (no staining of trichomes) | - | - (no staining of trichomes) | - | - | +++ |
| T447-21-6 | - (no staining of trichomes) | - | - (no staining of trichomes) | - | - | +++ |
| T447-22-2 | - (no staining of trichomes) | - | - (no staining of trichomes) | - | - | +++ |
| T447-56 | + (no staining of trichomes, some staining near cut edges) | - | + some staining of surface glands near cut edges, no staining of trichomes) | - | - | +++ |

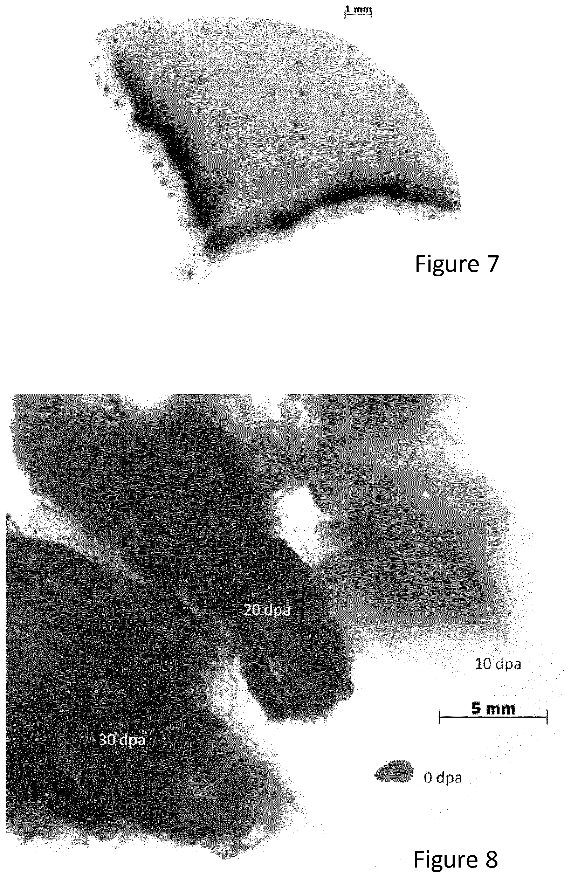

The Fb8-like (8K12) promoter also appears to be wound inducible as indicated by GUS staining around cut edges of leaves. A representative example is shown in Figure 7.

The Fb8-like(8K12) promoter appears to be highly fiber preferential although there is some expression in small glandular trichomes on other parts of the plant possible during the later stages of development of those glands. The promoter is also wound inducible and this is consistent with the Fb8-like gene being involved in rapid cell wall growth and thickening involved in the various process of fiber expansion and maturation, secretary gland maturation and cell wall repair after wounding.

### Example 5: Expression of a construct comprising pFb8-like operably linked to GUS in T1 plants

T1 seeds from four independent primary transformants with high expression in 20 dpa fibre were collected and planted in the glasshouse. These lines were: T447-1-1, T447-21-6, T447-22-2 and T447-56. Similar staining patterns were observed in all four lines. Sections of cotyledons were stained with X-Gluc as previously described. Gus activity was observed around cut edges and along the vasculature leading away from the cut edges. Staining was also observed in some small glandular trichomes that are distributed across the cotyledon particularly along the vasculature. These reactions are typical of a wounding response and have also been observed in other tissues of the T1 plant including leaves and petioles. A representative example is shown in Figure 7.

Two T1 plants from each line were grown to maturity and flowers tagged to collect bolls of different ages (0, 10, 20 and 30 dpa) and various other tissues harvested including leaf, petiole, stem, root, bollcoat (at 10 and 20 dpa), small flower bud and various flower parts at 0 dpa (including flower bract, petal, stamens and stigma). These were stained for GUS activity. GUS staining was in X-Gluc solution overnight at 37°C as described previously. Tissues were cleared in an ethanol series until no more chlorophyll was extracted and photographed. All lines had similar staining patterns, but the staining pattern for line T-447-22-2 is summarized in Table 3, while figures 8 and 9 are pictures of fibers of different ages and of different cotton tissues in T1 plants of T-447-22-2.

**Table 3: Staining pattern in different cotton tissues of T1-plants of line T-447-22-2**

| Tissue | Comment |
|---|---|
| 0 dpa ovule | Ovule surface only very weakly stained |
| 10 dpa fiber and seed | Fibers unstained, seed coat moderately stained |
| 20 dpa fiber and seed | Fibers moderately stained, seed coat moderately stained |
| 30 dpa fiber | Fibers intensely stained, seed coat stained |
| Cotyledon | Some small glandular trichomes stained, stronger staining from cut edges mostly following the vasculature. No trichomes stained. |
| Leaf | Staining in veins near wound sites, no hair trichomes stained on leaves. |
| Leaf Petiole | Staining near cut ends, hair trichomes not stained |
| Stem | Weak staining throughout stem and in sub-epidermal glands. No hair trichomes stained |
| Root | No staining |
| Boll Coat (0, 10 and 20 dpa) | Moderate staining of the bollcoat septum separating locules and at some cut edges |
| Flower bud | Staining around the wound areas near the bracts and other wound sites and in an around ovules. |
| Flower Bract | Staining around some cut edges near the base of the bract, but not hair trichomes. |
| Petal | Some staining of vasculature near distal end of the petal. No staining near the base of the petal or in the trichomes around the base. |
| Stamen/Anther/Pollen | No staining in the anther wall, but weak staining in the filament. Strong staining in pollen |
| Stigma/Style | Staining around the tip of the stigma and at the base of the style where petals and bracts were removed. |

Overall, these data support that the Fb8-like 8K12 promoter is fiber-preferential and expressed throughout fiber development predominantly in older fibres undergoing secondary cell wall deposition. It is expressed elsewhere in the plant, but usually in a relatively small number of specialised cells or tissues, including small glandular trichomes or sub-epidermal glands on cotyledons, stems and petioles. The promoter appears to be wound responsiveness in a variety of tissues and is upregulated in the vasculature after wounding so may be responding to a hormonal signal transmitted through the vasculature.

### Example 6: Expression of chitin synthase under control of the 8K12 promoter in cotton

Chitin synthases can be expressed in cotton plants to increase positive charges in the cotton fiber by introducing chitin polymers into the fiber cell wall. For this, fiber-preferential or fiber-specific expression is important since plants transformed with a chitin synthase gene mostly do not show an appreciable phenotype if the promoter controlling expression of the chitin synthase is driving expression in many other tissues or cell types than fiber cells.

The following T-DNA vector comprising chimeric genes according to the invention was generated: T-DNA vector (pTIB359) comprising a nucleic acid sequence encoding the *Neurospora crassa* chitin synthase 2 gene (SEQ ID NO: 11) comprising a Golgi-targeting signal from *Arabidopsis thaliana* (Pagny et al., 2003, SEQ ID NO: 12) under control of the 8K12 promoter and further comprising a recombinant epsps gene as selectable marker gene, as well as a recombinant gene comprising the gfa (glutamine:fructose-6-phosphate amidotransferase) coding region from *E. coli* (Frohberg and Essigmann, 2006; SEQ ID NO: 13) under control of the 8K12 promoter The nucleotide sequence of this vector is represented in SEQ ID No. 14. The genetic elements of the vector are represented in Table 4.

**Table 4: Elements of pTIB359**

| Start | End | Name | Region |
|---|---|---|---|
| 1 | 25 | RB | Right border repeat from the T-DNA of Agrobacterium tumefaciens |
| 31 | 60 | MCS | Polylinker cloning sites |
| 61 | 1018 | Pfb8-lke-2 | sequence including the promoter region of the fb8-like gene (8K12) of Gossypium hirsutum |
| 1020 | 1124 | RPxylTAt | coding sequence for the Golgi retention peptide of the beta-1,2-xylosyltransferase gene of Arabidopsis thaliana (Pagny et al., 2003) |
| 1125 | 3956 | chs2Nc-1Pc | coding sequence of the chitin synthase 2 gene of Neurospora crassa |
| 3969 | 4205 | 3'35S | sequence including the 3' untranslated region of the 35S transcript of the Cauliflower Mosaic Virus |
| 4207 | 4242 | MCS | Polylinker cloning sites |
| 4243 | 5201 | Pfb8-lke-2 | sequence including the promoter region of the fb8-like gene of Gossypium hirsutum (cotton)(8K12) |
| 5202 | 7031 | gfaEc-1Pb | coding region of the glutamine:fructose-6-phosphate amidotransferase gene of Escherichia coli (Frohberg and Essigmann, 2006), adapted to plant codon usage |
| 7032 | 7045 | MCS | Polylinker cloning sites |
| 7049 | 7709 | 3'histonAt | sequence including the 3' untranslated region of the histone H4 gene of Arabidopsis thaliana |
| 7760 | 8674 | Ph4a748 ABC | sequence including the promoter region of the histone H4 gene of Arabidopsis thaliana |
| 8725 | 9187 | intronl h3At | first intron of gene II of the histone H3.III variant of Arabidopsis thaliana |
| 9194 | 9565 | TPotp C | coding sequence of the optimized transit peptide, containing sequence of the RuBisCO small subunit genes of Zea mays (corn) and Helianthus annuus (sunflower) |
| 9566 | 10903 | 2mepsps | coding sequence of the double-mutant 5-enol-pyruvylshikimate-3-phosphate synthase gene of Zea mays (corn) |
| 10927 | 11587 | 3'histon At | sequence including the 3' untranslated region of the histone H4 gene of Arabidopsis thaliana ( |
| 11686 | 11724 | LB | Left border repeat from the T-DNA of Agrobacterium tumefaciens |
| 13585 | 12031 C | aadA | Sm/Sp R - the coding sequence of the aminoglycoside adenyltransferase gene (aadA) of Escherichia coli. |
| 13815 | 16604 | ORI pVS1 | Plasmid replication origin from pVS1 for stable maintenance in Agrobacterium. |
| 16605 | 17668 | ORI ColE1 | fragment including the origin of replication of the plasmid pBR322 for replication in Escherichia coli |

This vector was transferred into an appropriate Agrobacterium strain which was used for transforming cotton variety Coker312-17. 17 T0 lines were obtained which set seed. Field trials with progeny of at least one of these transgenic lines demonstrated that transgenic plants could be obtained with a phenotype almost identical to the wild type control.

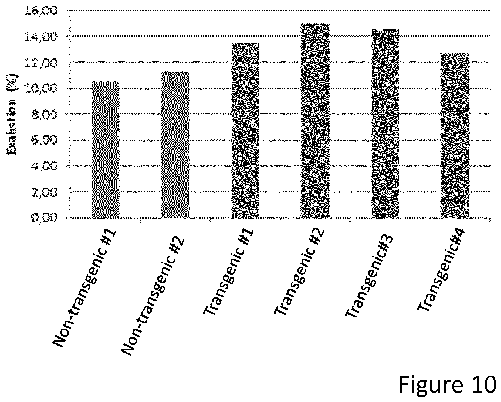

When cotton fibers harvested from greenhouse-grown plants of the above mentioned at least one transgenic line were dyed with Acid Orange 7, the transgenic lines resulted in a greater exhaustion of the remaining dye (13.9 for fibers from transgenic plants vs. 10.9 for fibers from non-transgenic controls; ie an increase of 27% exhaustion). These results are represented in Figure 10.

### References

Al-Ghazi Y, Bourot S, Arioli T, Dennis ES, Llewellyn DJ (2009) Transcript profiling during fiber development identifies pathways in secondary metabolism and cell wall structure that may contribute to cotton fiber quality. Plant and Cell Physiology 50: 1364-1381.
Carrington & Freed 1990. Cap-independent enhancement of translation by a plant potyvirus 5' nontranslated region. J. Virology 64, p. 1590-1597.
Chaubet et al. (1992). Genes encoding a histone H3.3-like variant in Arabidopsis contain intervening sequences. J Molecular Biology 225(2):569-74.
Cornelissen and Vandewiele (1989). Both RNA level and translation efficiency are reduced by anti-sense RNA in transgenic tobacco. Nucleic Acids Research 17(3):833-43.
Delaney et al. (2007). The fiber specificity of the cotton FSltp4 gene promoter is regulated by an AT-rich promoter region and the AT-hook transcription factor GhAT1. Plant and Cell Physiology 48, 1426-1437.
Eisner, T. et al. (1998). When defence backfires: detrimental effect of a plant's protective trichomes on an insect beneficial to the plant. Proceeding of the National Academy of Sciences, USA 95, p. 4410-4414.
Frohberg and Essigmann (2006) WO2006/032538.
Hsu et al. (1999). Analysis of promoter activity of cotton lipid transfer protein gene LTP6 in transgenic tobacco plants. Plant Science 143, p. 63-70.
Jefferson, R.A., Kavanagh, T.A. and Bevan, M.W. (1987) GUS fusions: β - glucuronidase as a sensitive and versatile gene fusion marker in higher plants. EMBO J. 6: 3901-3907.
John and Keller (1996). Metabolic pathway engineering in cotton: biosynthesis of polyhydroxybutyrate in fiber cell. Proceeding of the National Academy of Sciences, USA 93, 12678-12773.
Kim and Triplett, 82001). Cotton fiber growth in planta and in vitro. Models for plant cell elongation and cell wall biogenesis. Plant Physiology 127, p. 1361-1366.
Lazo, G.R., Stein, P.A., and Ludwig, R.A. (1991) A DNA transformation-competent Arabidopsis genomic library in Agrobacterium. Biotechnology 9: 963-967.
Li et al. (2005). The cotton ACTIN1 gene is functionally expressed in fibers and participates in fiber elongation. The Plant Cell 17, p. 859-875.
Lindbo, J.A. (2007). High-efficiency protein expression in plants from agroinfection-compatible Tobacco mosaic virus expression vectors. BMC Biotechnology 2007 Aug 27;7:52.
Liu et al. (2000). Cloning and promoter analysis of the cotton lipid transfer protein gene Ltp3. Biochimica et Biophysica Acta 1487, p. 106-111.
Liu, D., Zhang, X., Tu, L., Zhu, L., Guo X. (2006) Isolation by suppression-subtractive hybridization of genes preferentially expressed during early and late fiber development stages in cotton. Molecular Biology 40: 741-749.
Luo et al. (2007). GhDET2, a steroid 5a-reductase, plays an important role in cotton fiber cell initiation and elongation. The Plant Journal 51, p. 419-430.
Medeiros and Tingey, (2006). Glandular trichomes of Solanum berthaultii and its hybrids with Solanum tuberosum affect nymphal emergence, development, and survival of Empoasca fabae (Homoptera: Cicadellidae). Journal of Economic Entomology 99, p. 1483-1489.
Murray, F., Llewellyn, D., McFadden, H., Last, D., Dennis, E.S. and Peacock, W.J. (1999). Expression of the Talaromyces flavus glucose oxidase gene in cotton and tobacco reduces fungal infection, but is also phytotoxic. Molecular Breeding 5: 219-232.
Needleman and Wunsch (1970). A general method applicable to the search for similarities in the amino acid sequence of two proteins. J. Molecular Biology, 48, p.443-453.
S. Pagny, F. Bouisonnie, M. Sarkar, M.L. Follet-Gueye, A. Driouich, H. Schachter, L. Faye and V. Gomord (2003) Structural requirements for Arabidopsis β1,2-xylosyltransferase activity and targeting to the Golgi. The Plant Journal. Vol 33: 189-203
Pearson and Lipman (1988). Improved tools for biological sequence comparison. Proceeding of the National Academy of Sciences, USA 85, p.2444-48.
Ranger and Hower, 2001. Role of the glandular trichomes in resistance of perennial alfalfa to the potato leafhopper (Homoptera: Cicadellidae). Journal of Economic Entomology 94, p. 950-957.
Ruan et al. (2003). Suppression of sucrose synthase gene expression represses cotton fiber cell initiation, elongation, and seed development. The Plant Cell 15, p. 952-964.
Sambrook, J.F., Russell, D.W. and Irwin, N. (2000). Molecular Cloning: A Laboratory Manual, 3rd edition Volumes 1, 2, and 3. Cold Spring Harbor Laboratory Press.
Schünmann PHD, Llewellyn D, Surin B, Boevink P, Defeyter RC, Waterhouse PM (2003) A suite of novel promoters and terminators for plant biotechnology. Functional Plant Biology 30: 443-452.
Shangguan et al. (2008). Promoter of a cotton fiber MYB gene functional in trichomes of Arabidopsis and glandular trichomes of tobacco. Journal of Experimental Botany 59(13), p. 3533-3542.
Siomi, H. and Siomi, M. (2009). On the road to reading the RNA-interference code. Nature 457, 396-404.
Song et al. (2000). Expression of two tissue-specific promoters in transgenic cotton plants. Journal of Cotton Science 4, p. 217-223.
Szymanski et al. (2000). Progress in the molecular genetic analysis of trichome initiation and morphogenesis in Arabidopsis. Trends in Plant Science 5, p. 214-219.
Wagner et al. (2004). New approaches for studying and exploiting an old protuberance, the plant trichome. Annals of Botany 93, p. 3-11.
Wan CY, Wilkins TA (1994) A modified hot borate method significantly enhances the yield of high-quality RNA from cotton (Gossypium hirsutum L.). Analytical Biochemistry 223: 7-12.
Wang, S. et al. (2004). Control of plant trichome development by a cotton fiber MYB gene. The Plant Cell 16, p. 2323-2334.
Waterman, M. S. (1995). Introduction to Computational Biology: Maps, sequences and genomes. Chapman & Hall. London.
Werker, E. (2000). Trichome diversity and development. Advances in Botanical Research 31, p. 1-35.

## Claims

1. A nucleic acid comprising a nucleotide sequence selected from the group of
(a) at least 700 consecutive nucleotides of the nucleotide sequence set forth as SEQ ID NO: 1;
(b) the nucleotide sequence of SEQ ID No. 1 from the nucleotide at position 1 to the nucleotide at position 922;
(c) a nucleotide sequence having at least 95% sequence identity to the nucleic acid sequence of (a) or (b); and
wherein the nucleic acid has fiber-preferential promoter activity in cotton between 15 and 35 days post anthesis (dpa) and is not expressed in 10 dpa fibers, and wherein said nucleic acid having fiber-preferential promoter activity is not longer than 959 nucleotides.

2. A recombinant gene comprising
(a) the nucleic acid of claim1 operably linked to
(b) a heterologous nucleic acid encoding an expression product of interest, and optionally
(c) a transcription termination and polyadenylation region.

3. The recombinant gene of claim 2, wherein said expression product of interest is:
(a) a protein or an RNA molecule capable of modulating the expression of a gene endogenous to said plant; or
(b) a reporter gene or a fiber-specific gene.

4. The recombinant gene of claim 3, wherein said RNA molecule comprises a first and second RNA region wherein
(a) said first RNA region comprises a nucleotide sequence of at least 19 consecutive nucleotides having at least about 94% sequence identity to the nucleotide sequence of said endogenous gene;
(b) said second RNA region comprises a nucleotide sequence complementary to said 19 consecutive nucleotides of said first RNA region; and
(c) said first and second RNA region are capable of base-pairing to form a double stranded RNA molecule between at least said 19 consecutive nucleotides of said first and second region.

5. The recombinant gene of claim 2, wherein said heterologous nucleic acid encoding an expression product of interest encodes chitin synthase or glutamine:fructose-6-phosphate amidotransferase.

6. A transgenic cotton plant cell comprising a recombinant gene according to any one of claims 2 to 5.

7. A transgenic cotton plant comprising the recombinant gene of any one of claims 2 to 5 stably integrated in its genome or consisting of the transgenic cotton plant cells of claim 6.

8. The transgenic plant of claim 7 which is *G. hirsutum, G. barbadense, G. arboreum* or *G. herbaceum.*

9. A seed generated from a transgenic plant according to claim 7, wherein the seed comprises a recombinant gene according to claim 2.

10. Cotton fibers obtainable from the transgenic plant of claim 7.

11. A method of producing a transgenic cotton plant comprising
(a) providing a recombinant gene according to claim 2; and
(b) introducing said recombinant gene in a plant.

12. A method of effecting fiber-preferential expression of a product in cotton between 15 and 35 days post anthesis (dpa) but not in 10 dpa fibers comprising
(a) introducing the recombinant gene of claim 2 into the genome of a cotton plant; or
(b) providing the transgenic plant of claim 7.

13. A method of altering fiber properties in a cotton plant comprising
(a) introducing the recombinant gene of claim 2 into the genome of a cotton plant; or
(b) providing the transgenic plant of claim 7.

14. Use of the recombinant gene of claim 2 or the transgenic plant of claim 7 for fiber-preferential expression of a product in cotton between 15 and 35 days post anthesis (dpa) but not in 10 dpa fibers, for altering fiber properties in cotton or for increasing cotton yield.

## Patentansprüche

1. Nukleinsäure, umfassend eine Nukleotidsequenz, ausgewählt aus der folgenden Gruppe:
(a) mindestens 700 aufeinanderfolgende Nukleotide der Nukleotidsequenz gemäß SEQ ID NO: 1;
(b) der Nukleotidsequenz von SEQ ID NO: 1 vom Nukleotid an Position 1 bis zum Nukleotid an Position 922;
(c) einer Nukleotidsequenz mit mindestens 95% Sequenzidentität zu der Nukleinsäuresequenz gemäß (a) oder (b); und
wobei die Nukleinsäure faserpräferentielle Promoteraktivität in Baumwolle zwischen 15 und 35 Tagen nach der Blüte (days post anthesis, dpa) aufweist und in 10-dpa-Fasern nicht exprimiert wird, und wobei die Nukleinsäure mit faserpräferentieller Promoteraktivität nicht länger als 959 Nukleotide ist.

2. Rekombinantes Gen, umfassend
(a) die Nukleinsäure nach Anspruch 1 in operativer Verknüpfung mit
(b) einer heterologen Nukleinsäure, die für ein Expressionsprodukt von Interesse codiert, und gegebenenfalls
(c) eine Transkriptionsterminations- und Polyadenylierungsregion.

3. Rekombinantes Gen nach Anspruch 2, wobei es sich bei dem Expressionsprodukt von Interesse um Folgendes handelt:
(a) ein Protein oder ein RNA-Molekül, das fähig ist, die Expression eines in Bezug auf die Pflanze endogenen Gens zu modulieren; oder
(b) ein Reportergen oder ein faserspezifisches Gen.

4. Rekombinantes Gen nach Anspruch 3, wobei das RNA-Molekül eine erste und eine zweite RNA-Region umfasst, wobei
(a) die erste RNA-Region eine Nukleotidsequenz von mindestens 19 aufeinanderfolgenden Nukleotiden mit mindestens ungefähr 94% Sequenzidentität zu der Nukleotidsequenz des endogenen Gens umfasst;
(b) die zweite RNA-Region eine Nukleotidsequenz umfasst, die zu den 19 aufeinanderfolgenden Nukleotiden der ersten RNA-Region komplementär ist; und
(c) die erste und zweite RNA-Region zur Basenpaarung befähigt sind, wodurch ein doppelsträngiges RNA-Molekül zwischen zumindest den 19 aufeinanderfolgenden Nukleotiden der ersten und der zweiten Region gebildet wird.

5. Rekombinantes Gen nach Anspruch 2, wobei die heterologe Nukleinsäure, die für ein Expressionsprodukt von Interesse codiert, für die Chitinsynthase oder Glutamin:Fruktose-6-Phosphatamidotransferase codiert.

6. Transgene Baumwollpflanzenzelle, umfassend ein rekombinantes Gen nach einem der Ansprüche 2 bis 5.

7. Transgene Baumwollpflanze, umfassend das rekombinante Gen nach einem der Ansprüche 2 bis 5 stabil in ihr Genom integriert oder bestehend aus den transgenen Baumwollpflanzenzellen nach Anspruch 6.

8. Transgene Pflanze nach Anspruch 7, bei der es sich um *G. hirsutum, G. barbadense, G. arboreum* oder *G. herbaceum* handelt.

9. Samen, produziert von einer transgenen Pflanze nach Anspruch 7, wobei der Samen ein rekombinantes Gen nach Anspruch 2 umfasst.

10. Baumwollfasern, erhältlich von der transgenen Pflanze nach Anspruch 7.

11. Verfahren zum Erzeugen einer transgenen Baumwollpflanze, umfassend
(a) das Bereitstellen eines rekombinanten Gens nach Anspruch 2; und
(b) Einführen des rekombinanten Gens in eine Pflanze.

12. Verfahren zum Bewirken von faserpräferentieller Expression eines Produkts in Baumwolle zwischen 15 und 35 Tagen nach der Blüte (days post anthesis, dpa), jedoch nicht in 10-dpa-Fasern, das Folgendes umfasst:
(a) Einführen des rekombinanten Gens nach Anspruch 2 in das Genom einer Baumwollpflanze; oder
(b) Bereitstellen der transgenen Pflanze nach Anspruch 7.

13. Verfahren zur Veränderung der Fasereigenschaften in einer Baumwollpflanze, das Folgendes umfasst:
(a) Einführen des rekombinanten Gens nach Anspruch 2 in das Genom einer Baumwollpflanze; oder
(b) Bereitstellen der transgenen Pflanze nach Anspruch 7.

14. Verwendung des rekombinanten Gens nach Anspruch 2 oder der transgenen Pflanze nach Anspruch 7 für die faserpräferentielle Expression eines Produkts in Baumwolle zwischen 15 und 35 Tagen nach der Blüte (days post anthesis, dpa), jedoch nicht in 10-dpa-Fasern, zum Verändern der Fasereigenschaften in Baumwolle oder zum Erhöhen des Baumwollertrags.

## Revendications

1. Acide nucléique comprenant une séquence nucléotidique choisie dans le groupe de
(a) au moins 700 nucléotides consécutifs de la séquence nucléotidique décrite dans SEQ ID NO: 1 ;
(b) la séquence nucléotidique de SEQ ID No. 1 du nucléotide à la position 1 au nucléotide à la position 922 ;
(c) une séquence nucléotidique ayant au moins 95 % d'identité de séquence avec la séquence d'acide nucléique de (a) ou (b) ; et
l'acide nucléique ayant une activité de promoteur préférentiel dans les fibres dans du coton entre 15 et 35 jours post-anthèse (jpa) et n'étant pas exprimé dans des fibres à 10 jpa, et ledit acide nucléique ayant une activité de promoteur préférentiel dans les fibres n'étant pas plus long que 959 nucléotides.

2. Gène recombinant comprenant
(a) l'acide nucléique selon la revendication 1 fonctionnellement lié à
(b) un acide nucléique hétérologue codant pour un produit d'expression d'intérêt, et facultativement
(c) une région de terminaison de transcription et de polyadénylation.

3. Gène recombinant selon la revendication 2, dans lequel ledit produit d'expression d'intérêt est :
(a) une protéine ou une molécule d'ARN capable de moduler l'expression d'un gène endogène à ladite plante ; ou
(b) un gène rapporteur ou un gène spécifique de fibre.

4. Gène recombinant selon la revendication 3, dans lequel ladite molécule d'ARN comprend une première et une deuxième région d'ARN, dans lequel
(a) ladite première région d'ARN comprend une séquence nucléotidique d'au moins 19 nucléotides consécutifs ayant au moins environ 94 % d'identité de séquence avec la séquence nucléotidique dudit gène endogène ;
(b) ladite deuxième région d'ARN comprend une séquence nucléotidique complémentaire desdits 19 nucléotides consécutifs de ladite première région d'ARN ; et
(c) lesdites première et deuxième régions d'ARN sont capables d'appariement de bases pour former une molécule d'ARN double brin entre au moins lesdits 19 nucléotides consécutifs desdites première et deuxième régions.

5. Gène recombinant selon la revendication 2, dans lequel ledit acide nucléique hétérologue codant pour un produit d'expression d'intérêt code pour la chitine synthase ou la glutamine:fructose-6-phosphate amidotransférase.

6. Cellule de plante de coton transgénique comprenant un gène recombinant selon l'une quelconque des revendications 2 à 5.

7. Plante de coton transgénique comprenant le gène recombinant selon l'une quelconque des revendications 2 à 5 intégré de façon stable dans son génome ou constitué des cellules de plante de coton transgénique selon la revendication 6.

8. Plante transgénique selon la revendication 7 qui est *G. hirsutum, G. barbadense, G. arboreum* ou *G. herbaceum.*

9. Graine générée à partir d'une plante transgénique selon la revendication 7, la graine comprenant un gène recombinant selon la revendication 2.

10. Fibres de coton pouvant être obtenues à partir de la plante transgénique selon la revendication 7.

11. Procédé de production d'une plante de coton transgénique comprenant
(a) la fourniture d'un gène recombinant selon la revendication 2 ; et
(b) l'introduction dudit gène recombinant dans une plante.

12. Procédé de conduite de l'expression préférentielle dans les fibres d'un produit dans du coton entre 15 et 35 jours post-anthèse (jpa) mais pas dans des fibres à 10 jpa comprenant
(a) l'introduction du gène recombinant selon la revendication 2 dans le génome d'une plante de coton ; ou
(b) la fourniture de la plante transgénique selon la revendication 7.

13. Procédé de modification des propriétés de fibre dans une plante de coton comprenant
(a) l'introduction du gène recombinant selon la revendication 2 dans le génome d'une plante de coton ; ou
(b) la fourniture de la plante transgénique selon la revendication 7.

14. Utilisation du gène recombinant selon la revendication 2 ou de la plante transgénique selon la revendication 7 pour l'expression préférentielle dans les fibres d'un produit dans du coton entre 15 et 35 jours post-anthèse (jpa) mais pas dans des fibres à 10 jpa, pour modifier les propriétés de fibre dans du coton ou pour augmenter le rendement en coton.


### SEQUENCE LISTING

<110> Bayer CropScience NV
   Commonwealth Scientific and Industrial Research Organisation
<120> Novel fiber-preferential promoter in cotton
<130> BCS 13-2022
<160> 14
<170> PatentIn version 3.5
<210> 1
   <211> 959
   <212> DNA
   <213> Artificial Sequence
<220>
   <223> fiber-selective promoter region from BAC8K12
<400> 1
   aagcttaaaa tcacttatca atttcaaaaa cagaggttag ccgaatgcta agagcttaaa      60
   aatggcttct tttgtttctt tttcttgcaa acggtggaga gaagagggaa atgaagattg     120
   accatatctt tttttattat gttttaacat ataatattaa taatttaatc ataattatac     180
   tttggtgaat gtgacagtgg ggatatacgt aaagtatata acattatact ttttgcaagc     240
   agttggctgg tctacccaag agtgatcaaa ttttgagctg ccttcaatga gccaattttt     300
   gcctataatg gataaaggca ctttgtctag ttcaactgct cacagaataa tgttaaaatg     360
   aaattaaaac aaggtgggct ggtcacacca aaaaaataaa atattaatgt ggtgtttggt     420
   tagtcgaatt tatcataatt ttatagaaat tgagactaaa aaacattaag agaataaatt     480
   ctataacaaa gacaatttag taaaaatgtc cttttaggta attttaagta ctcttaacca     540
   aaataaaaaa ttcaaatcaa attaaccaaa taagataata taacatacgg aacatcccac     600
   ttataatctt acatccccgt aatcttatta tgaaaagtaa tcttatatta ctcgaatcaa     660
   atgctctcac aaactattat ctaaataaag aaaaacactt aatttttata acattttttt     720
   tcatatattt gaaagattat attttgtatt tttacgtaaa aatatttgac atagattgag     780
   caccttttta acataattcc accataagtc aattatgtag atgagaaatt ggtacaaaca     840
   acgtggagcc aaatcccacc aaaccatctc tcatcctctc ctataaaagg cttgctacac     900
   atagacaaca atccacacaa aaatacactt aaaattcttt tctttctatt tggttaacc      959
<210> 2
   <211> 28
   <212> DNA
   <213> Artificial Sequence
<220>
   <223> oligonucleotide primer Fb8-like_F
<400> 2
   attcttttct ttctatttgg ttaaccat 28
<210> 3
   <211> 20
   <212> DNA
   <213> Artificial Sequence
<220>
   <223> oligonucleotide primer FB8-like_R
<400> 3
   tagtgctcga gccagactga 20
<210> 4
   <211> 20
   <212> DNA
   <213> Artificial Sequence
<220>
   <223> oligonucleotide primer QFb81ike_F
<400> 4
   gagtgccaag agtcacacga 20
<210> 5
   <211> 20
   <212> DNA
   <213> Artificial Sequence
<220>
   <223> oligonucleotide primer QFb8like_R
<400> 5
   ttggatccca cccttaaaca 20
<210> 6
   <211> 20
   <212> DNA
   <213> Artificial Sequence
<220>
   <223> oligonucleotide primer UbiQ_F
<400> 6
   aatccacttt gcacctggtc 20
<210> 7
   <211> 20
   <212> DNA
   <213> Artificial Sequence
<220>
   <223> oligonucleotide primer UbiQ-R
<400> 7
   ctagtggcca gctcacatca 20
<210> 8
   <211> 63
   <212> DNA
   <213> Artificial Sequence
<220>
   <223> oligonucleotide sequence gwFb8-likePro(8K12)_F
<400> 8
   ggggacaagt ttgtacaaaa aagcaggcta agcttaaaat cacttatcaa tttcaaaaac 60
   aga 63
<210> 9
   <211> 62
   <212> DNA
   <213> Artificial Sequence
<220>
   <223> oligonucleotide primer gwFb8-likePro(8K12)_R
<400> 9
   ggggaccact ttgtacaaga aagctgggtg gttaaccaaa tagaaagaaa agaattttaa 60
   gt 62
<210> 10
   <211> 1943
   <212> DNA
   <213> Artificial Sequence
<220>
   <223> promoter and gene for BAC8K12
<400> 10
   aagcttaaaa tcacttatca atttcaaaaa cagaggttag ccgaatgcta agagcttaaa 60
   aatggcttct tttgtttctt tttcttgcaa acggtggaga gaagagggaa atgaagattg 120
   accatatctt tttttattat gttttaacat ataatattaa taatttaatc ataattatac 180
   tttggtgaat gtgacagtgg ggatatacgt aaagtatata acattatact ttttgcaagc 240
   agttggctgg tctacccaag agtgatcaaa ttttgagctg ccttcaatga gccaattttt 300
   gcctataatg gataaaggca ctttgtctag ttcaactgct cacagaataa tgttaaaatg 360
   aaattaaaac aaggtgggct ggtcacacca aaaaaataaa atattaatgt ggtgtttggt 420
   tagtcgaatt tatcataatt ttatagaaat tgagactaaa aaacattaag agaataaatt 480
   ctataacaaa gacaatttag taaaaatgtc cttttaggta attttaagta ctcttaacca 540
   aaataaaaaa ttcaaatcaa attaaccaaa taagataata taacatacgg aacatcccac 600
   ttataatctt acatccccgt aatcttatta tgaaaagtaa tcttatatta ctcgaatcaa 660
   atgctctcac aaactattat ctaaataaag aaaaacactt aatttttata acattttttt 720
   tcatatattt gaaagattat attttgtatt tttacgtaaa aatatttgac atagattgag 780
   caccttttta acataattcc accataagtc aattatgtag atgagaaatt ggtacaaaca 840
   acgtggagcc aaatcccacc aaaccatctc tcatcctctc ctataaaagg cttgctacac 900
   atagacaaca atccacacaa aaatacactt aaaattcttt tctttctatt tggttaacca 960
   tggctcataa ctttcgtcat cctttcttcc ttttccaact tttactcatt actgtctcac 1020
   taatgatcgg tagccacacc gtctcgtcag cggctcgaca tttattccac acacaaacaa 1080
   cctcatcaga gctgccacaa ttggcttcaa aatacgaaaa gcacgaagag tctgaataca 1140
   aacagccaaa atatcatgaa gagtacccaa aacatgagaa gcctgaaatg tacaaggagg 1200
   aaaaacaaaa accctgcaaa catcatgaag agtaccacga gtcacgcgaa tcgaaggagc 1260
   acgaagagta cgataaagaa aaacccgatt tccccaaatg ggaaaagcct aaagagcacg 1320
   agaaacacga agtcgaatat ccgaaaatac ccgagtacaa ggacaaacaa gatgaggata 1380
   aggaacataa aaatgaagag taccacgaat cacgtgaatc gaaggagcac gaagaatacg 1440
   agaaagaaaa acccgagttc cccaaacggg aaaagcctaa agagcacgag aaacacgaag 1500
   tcgaatattc ggaaataccc gagtacaagg aaaaggaaga taagagtaag aaacataaag 1560
   atgaagagtg ccaggagtca cacgaatcga aagagcacga agagtacgag aaagaaaaac 1620
   ccgatttccc caaatgggaa aagcctaaag ggcacgagaa acataaagcc gaatatccga 1680
   aaatacctga gtgcaaggaa aaactagatg aggataagga acataaacat gagttcccaa 1740
   agcatgaaaa agaagaggag aagaaacctg agaaaggcat agtaccctga gtgggttaaa 1800
   atgcctgaat ggccgaagtc catgtttact cagtctggct cgagcactaa gccttaagcc 1860
   atatgacact ggtgcatgtg ccatcatcat gcagtaattt catgggatat cgtaattata 1920
   ttgttaataa aaagatgtga gtg 1943
<210> 11
   <211> 2831
   <212> DNA
   <213> Neurospora crassa
<400> 11
   agtccagaat cagcaaccgg ttatcgagtt ccgccacaag gacggtacga gccttcagaa        60
   atcgatgtca tgccaggcca gggacaccgg gatcgagtta cggaaatgcg aggcgaccgc       120
   ttccctcggc accagcgcct ttacactaca atagcccaag tcgcgcagcg agtcattatc       180
   cacggtacca tggaggttat gcggacgacg tgacagttag catgggaccg gacgacgatc       240
   gtacagatat ctttggcccc gaaaccgatc tcagcgaaac gcgccacctc aacgacgcat       300
   acgggtttcg gtcatcccag atcaccctca gcgaagatcc ccacggcacc cacgcgcgtt       360
   cccggtacga cgacgaagac gatgtgagca ccacttattc ctccaacacg ggcaccagcg       420
   cttcaggtgt cgacaagttc gagcattacg gtcccattcc ggaggaaggc aagcacgagc      480
   ggcgcggcgt gcgaccacca cagatgtcga ggaaggaagt ccagctcatc aacggcgaac      540
   tcgttctcga gtgcaagatt ccgactatat tgtattcgtt tttgcccagg agagacgaag      600
   tggagtttac gcacatgcgg tacacagccg tcacttgtga ccctgatgac tttgttgcca      660
   ggggttacaa gttgcgccag aatatcggtc gtaccgccag ggagacggag ctgttcatct      720
   gcgtgaccat gtacaacgag gacgagttcg gattcacacg gactatgcac gcagtgatga      780
   agaacatttc gcatttttgt tcccgaaaca agagtaggac gtggggagcg gatgggtggc      840
   agaagattgt ggtctgtgtg gtttcggatg gacgagagat cattcacccc cggaccttgg      900
   acgccctcgc agccatgggc gtttaccagc acggtatcgc caagaacttt gtcaaccaga      960
   aggcggtgca ggcccacgtt tacgagtaca cgacacaagt gtctctggac agcgacctca     1020
   agttcaaggg cgccgagaag ggcatcgtgc cctgccagat gattttttgc ttgaaggaga      1080
   agaaccaaaa gaaactcaac tcgcatagat ggttcttcaa cgcctttggc aaagccttga     1140
   acccgaatgt gtgtatcctc ctagacgtcg gcacccgccc cggcggcaca agtctctacc     1200
   atctctggaa agccttcgac acggattcca acgtggcggg ggcctgcggg gaaatcaaag     1260
   cgatgaaggg gcggtttggc gggaatttgc tcaaccctct ggtggctagt cagaactttg     1320
   agtacaagat gagcaatatt ctggacaaac cgttggagtc ggtgtttggg tacatcacgg     1380
   tgttgccggg cgccttgtcg gcgtatcggt accatgcgct gcagaacgat gagacgggcc     1440
   atgggccgtt gagtcagtat ttcaagggcg agacgctcca tgggcagcac gcggatgtgt     1500
   ttacggcgaa catgtacttg gccgaggacc gaattctgtg ttgggagttg gtggccaaga     1560
   ggggtgagag gtgggtgttg aagtatgtga aggggtgtac gggtgagacg gatgtgcctg     1620
   acaccgtccc ggaattcgtc tcgcaacgtc gtcgttggct caacggtgcc ttcttcgccg     1680
   ccgtctactc cctcgtccac tttcgacaaa tctggaaaac cgaccacacc tttatgcgca     1740
   aagcccttct ccacgtcgaa ttcctctacc acctcctgca actcctcttc acctacttct     1800
   ccctggccaa cttctacctc gccttctact ttatcgccgg cggactcgcc gatccccacg     1860
   tcgacccttt taactcggac ggccacgtcg cgcgcatcat cttcaacatc ctccgctacg     1920
   tctgcgtcct gctgatctgc acacaattca tcttgtccct cggcaaccgt ccgcagggtg     1980
   ccaaaagaat gtatctcgca tccatgatca tctacgccgt catcatggtg tacaccacct     2040
   tcgccaccat cttcatcgtc gtgcgacaaa tccaaccctc tcaaaaatcc gacgacaagc     2100
   ccgacctcga actcggcaac aacgtcttca ccaacctgat cgtctccgtg gctagtaccc     2160
   tcgggctcta cttcgtcatg tcctttctct atctcgaccc ctggcacatg ttcacctcgg     2220
   ccatccagta ctttgtcctg ctgccttcct acatctgcac gctccagatc tacgcctttt     2280
   gcaacaccca cgacgtcaca tggggcacca aaggcgacaa cgtgatgcgc accgatctcg     2340
   gaggcgccat tggcaaggga agcaccgtcg aactggaaat gccttcggac caactcgaca 2400
   tcgactcggg atacgacgaa tgtctacgaa atctccggga tcgcgtcatg gtccctgccg 2460
   ttcccgtgtc cgaggaccag ctgcagcagg attactacaa gtcggtgcgc acgtacatgg 2520
   tggtgtcgtg gatggtggcc aacgcgacgc tggccatggc ggtgtcggaa gcgtatggcg 2580
   attcggaaat tggggataat ttttacttgc ggtttatcct gtgggcggtg gcggccctgg 2640
   cgctgtttag agcgttgggg tcgacgacgt ttgcggcgat taatctggtg agtgctctcg 2700
   tggagggcag ggtcaggctg aggttgaata tgaaagggtt taggtggatt aaggagaagt 2760
   ggggggatgc ggatgtgaag ggcaagtttg aggggttggg ggatcgggcg agggggttgg 2820
   cgaggcggtg a 2831
<210> 12
   <211> 105
   <212> DNA
   <213> Arabidopsis thaliana
<400> 12
   atgagtaaac ggaatccgaa gattctgaag atttttctgt atatgttact tctcaactct 60
   ctctttctca tcatctactt cgtttttcac tcatcgtcgt tttca 105
<210> 13
   <211> 1830
   <212> DNA
   <213> Escherichia coli
<400> 13
   atgtgcggaa ttgttggtgc tatcgcccaa agagacgttg ctgagatttt gttagagggt      60
   ctgcgaaggc tagagtatag aggatatgac tccgctggtc tggctgtcgt tgatgctgag     120
   ggtcatatga caaggctaag aaggttagga aaggttcaga tgcttgctca ggcagctgag     180
   gaacatccat tgcatggagg tactggtatt gcacatacca ggtgggctac tcatggggag     240
   ccatcagaag ttaatgctca tccacatgtg agtgagcata tcgttgtagt tcacaatggg     300
   ataattgaaa accacgaacc attgagggaa gagttaaagg caagaggata tacttttgtg     360
   agtgagactg acactgaggt tattgcacat ttagtgaact gggaactcaa acaggggggc     420
   acattgcgtg aggctgtgtt aagagctatt cctcaactta gaggtgcata cggtactgtt     480
   attatggatt caagacaccc agatactctc cttgcagcta gatcaggtag tcccttggtc     540
   ataggacttg gaatgggtga aaattttatc gctagcgacc aattggcctt attgccagtt     600
   acaagacgat ttattttcct tgaagagggc gatattgctg agattactag aaggtctgtg     660
   aacatctttg ataagactgg cgctgaggtt aaacgtcagg atatcgagtc taaccttcaa     720
   tacgatgctg gtgataaagg aatttacagg cattatatgc aaaaggaaat ttatgaacaa     780
   ccaaatgcta tcaaaaacac acttactggc cgtatttctc atggacaggt cgatttaagc     840
   gagcttggtc ctaatgcaga cgaactgcta tcaaaagttg agcacataca gatactggca     900
   tgcggaacta gttataattc aggaatggtg tctagatact ggttcgaaag cttggcaggt     960
   ataccttgtg atgtagagat cgcttctgag tttaggtata gaaagtctgc tgtgcgtaga    1020
   aattcattaa tgattacatt atctcaatcc ggagaaacag cagatacact ggctggattg    1080
   aggctttcta aggaactcgg atatctgggt tcacttgcta tttgtaatgt accaggttcc    1140
   tcattggttc gtgaatcaga tctagcactt atgacaaatg caggaactga aataggtgtg    1200
   gcaagtacca aggctttcac aacccaactg accgtacttt taatgttggt agcaaaactc    1260
   agtcgattaa aggggctaga tgcatctatc gaacatgata ttgttcacgg gcttcaagct    1320
   ctcccttcaa gaattgaaca aatgctttca caagataaga gaatagaggc attggctgaa    1380
   gatttttccg acaaacatca cgcattgttt cttggacgtg gcgatcaata tccaattgca    1440
   ttggaaggag ctttgaagtt gaaagaaata agttacattc acgcagaagc atatgcagct    1500
   ggagaactca agcatggtcc tttggcactc atcgacgctg acatgcccgt gatcgtagtg    1560
   gctcctaata acgaactgct cgaaaagctt aaatcaaata tcgaagaggt tcgagctaga    1620
   ggaggtcagc tttacgtttt cgctgaacaa gatgctggat tcgtgtcaag cgataatatg    1680
   catataattg aaatgcctca cgttgaagaa gtgattgcac ctatatttta tacagtccca    1740
   ttgcaacttc tagcttacca tgttgcactt attaaaggaa ctgatgttga tcagcctaga    1800
   aacctagcaa aatctgtaac agtcgaataa                                     1830
<210> 14
   <211> 17967
   <212> DNA
   <213> Artificial Sequence
<220>
   <223> pTIB359
<400> 14
   aattacaacg gtatatatcc tgccagtact gggccccctc gagggcgatc gcgcggccgc      60
   aagcttaaaa tcacttatca atttcaaaaa cagaggttag ccgaatgcta agagcttaaa     120
   aatggcttct tttgtttctt tttcttgcaa acggtggaga gaagagggaa atgaagattg     180
   accatatctt tttttattat gttttaacat ataatattaa taatttaatc ataattatac     240
   tttggtgaat gtgacagtgg ggatatacgt aaagtatata acattatact ttttgcaagc     300
   agttggctgg tctacccaag agtgatcaaa ttttgagctg ccttcaatga gccaattttt     360
   gcctataatg gataaaggca ctttgtctag ttcaactgct cacagaataa tgttaaaatg     420
   aaattaaaac aaggtgggct ggtcacacca aaaaaataaa atattaatgt ggtgtttggt     480
   tagtcgaatt tatcataatt ttatagaaat tgagactaaa aaacattaag agaataaatt     540
   ctataacaaa gacaatttag taaaaatgtc cttttaggta attttaagta ctcttaacca      600
   aaataaaaaa ttcaaatcaa attaaccaaa taagataata taacatacgg aacatcccac      660
   ttataatctt acatccccgt aatcttatta tgaaaagtaa tcttatatta ctcgaatcaa      720
   atgctctcac aaactattat ctaaataaag aaaaacactt aatttttata acattttttt      780
   tcatatattt gaaagattat attttgtatt tttacgtaaa aatatttgac atagattgag      840
   caccttttta acataattcc accataagtc aattatgtag atgagaaatt ggtacaaaca      900
   acgtggagcc aaatcccacc aaaccatctc tcatcctctc ctataaaagg cttgctacac      960
   atagacaaca atccacacaa aaatacactt aaaattcttt tctttctatt tggttaacca     1020
   tgagtaaacg gaatccgaag attctgaaga tttttctgta tatgttactt ctcaactctc     1080
   tctttctcat catctacttc gtttttcact catcgtcgtt ttcagagtcc agaatcagca     1140
   accggttatc gagttccgcc acaaggacgg tacgagcctt cagaaatcga tgtcatgcca     1200
   ggccagggac accgggatcg agttacggaa atgcgaggcg accgcttccc tcggcaccag     1260
   cgcctttaca ctacaatagc ccaagtcgcg cagcgagtca ttatccacgg taccatggag     1320
   gttatgcgga cgacgtgaca gttagcatgg gaccggacga cgatcgtaca gatatctttg     1380
   gccccgaaac cgatctcagc gaaacgcgcc acctcaacga cgcatacggg tttcggtcat     1440
   cccagatcac cctcagcgaa gatccccacg gcacccacgc gcgttcccgg tacgacgacg     1500
   aagacgatgt gagcaccact tattcctcca acacgggcac cagcgcttca ggtgtcgaca     1560
   agttcgagca ttacggtccc attccggagg aaggcaagca cgagcggcgc ggcgtgcgac     1620
   caccacagat gtcgaggaag gaagtccagc tcatcaacgg cgaactcgtt ctcgagtgca     1680
   agattccgac tatattgtat tcgtttttgc ccaggagaga cgaagtggag tttacgcaca     1740
   tgcggtacac agccgtcact tgtgaccctg atgactttgt tgccaggggt tacaagttgc     1800
   gccagaatat cggtcgtacc gccagggaga cggagctgtt catctgcgtg accatgtaca     1860
   acgaggacga gttcggattc acacggacta tgcacgcagt gatgaagaac atttcgcatt     1920
   tttgttcccg aaacaagagt aggacgtggg gagcggatgg gtggcagaag attgtggtct     1980
   gtgtggtttc ggatggacga gagatcattc acccccggac cttggacgcc ctcgcagcca      2040
   tgggcgttta ccagcacggt atcgccaaga actttgtcaa ccagaaggcg gtgcaggccc     2100
   acgtttacga gtacacgaca caagtgtctc tggacagcga cctcaagttc aagggcgccg     2160
   agaagggcat cgtgccctgc cagatgattt tttgcttgaa ggagaagaac caaaagaaac     2220
   tcaactcgca tagatggttc ttcaacgcct ttggcaaagc cttgaacccg aatgtgtgta     2280
   tcctcctaga cgtcggcacc cgccccggcg gcacaagtct ctaccatctc tggaaagcct     2340
   tcgacacgga ttccaacgtg gcgggggcct gcggggaaat caaagcgatg aaggggcggt     2400
   ttggcgggaa tttgctcaac cctctggtgg ctagtcagaa ctttgagtac aagatgagca 2460
   atattctgga caaaccgttg gagtcggtgt ttgggtacat cacggtgttg ccgggcgcct 2520
   tgtcggcgta tcggtaccat gcgctgcaga acgatgagac gggccatggg ccgttgagtc 2580
   agtatttcaa gggcgagacg ctccatgggc agcacgcgga tgtgtttacg gcgaacatgt 2640
   acttggccga ggaccgaatt ctgtgttggg agttggtggc caagaggggt gagaggtggg 2700
   tgttgaagta tgtgaagggg tgtacgggtg agacggatgt gcctgacacc gtcccggaat 2760
   tcgtctcgca acgtcgtcgt tggctcaacg gtgccttctt cgccgccgtc tactccctcg 2820
   tccactttcg acaaatctgg aaaaccgacc acacctttat gcgcaaagcc cttctccacg 2880
   tcgaattcct ctaccacctc ctgcaactcc tcttcaccta cttctccctg gccaacttct 2940
   acctcgcctt ctactttatc gccggcggac tcgccgatcc ccacgtcgac ccttttaact 3000
   cggacggcca cgtcgcgcgc atcatcttca acatcctccg ctacgtctgc gtcctgctga 3060
   tctgcacaca attcatcttg tccctcggca accgtccgca gggtgccaaa agaatgtatc 3120
   tcgcatccat gatcatctac gccgtcatca tggtgtacac caccttcgcc accatcttca 3180
   tcgtcgtgcg acaaatccaa ccctctcaaa aatccgacga caagcccgac ctcgaactcg 3240
   gcaacaacgt cttcaccaac ctgatcgtct ccgtggctag taccctcggg ctctacttcg 3300
   tcatgtcctt tctctatctc gacccctggc acatgttcac ctcggccatc cagtactttg 3360
   tcctgctgcc ttcctacatc tgcacgctcc agatctacgc cttttgcaac acccacgacg 3420
   tcacatgggg caccaaaggc gacaacgtga tgcgcaccga tctcggaggc gccattggca 3480
   agggaagcac cgtcgaactg gaaatgcctt cggaccaact cgacatcgac tcgggatacg 3540
   acgaatgtct acgaaatctc cgggatcgcg tcatggtccc tgccgttccc gtgtccgagg 3600
   accagctgca gcaggattac tacaagtcgg tgcgcacgta catggtggtg tcgtggatgg 3660
   tggccaacgc gacgctggcc atggcggtgt cggaagcgta tggcgattcg gaaattgggg 3720
   ataattttta cttgcggttt atcctgtggg cggtggcggc cctggcgctg tttagagcgt 3780
   tggggtcgac gacgtttgcg gcgattaatc tggtgagtgc tctcgtggag ggcagggtca 3840
   ggctgaggtt gaatatgaaa gggtttaggt ggattaagga gaagtggggg gatgcggatg 3900
   tgaagggcaa gtttgagggg ttgggggatc gggcgagggg gttggcgagg cggtgagcta 3960
   gcaagcttgg acacgctgaa atcaccagtc tctctctaca aatctatctc tctctatttt 4020
   ctccataata atgtgtgagt agttcccaga taagggaatt agggttccta tagggtttcg 4080
   ctcatgtgtt gagcatataa gaaaccctta gtatgtattt gtatttgtaa aatacttcta 4140
   tcaataaaat ttctaattcc taaaaccaaa atccagtact aaaatccaga tcatgcatgg 4200
   tacagcacgc gtcctgcagg cccgggttaa ttaagcggcc gcaagcttaa aatcacttat 4260
   caatttcaaa aacagaggtt agccgaatgc taagagctta aaaatggctt cttttgtttc 4320
   tttttcttgc aaacggtgga gagaagaggg aaatgaagat tgaccatatc tttttttatt 4380
   atgttttaac atataatatt aataatttaa tcataattat actttggtga atgtgacagt 4440
   ggggatatac gtaaagtata taacattata ctttttgcaa gcagttggct ggtctaccca 4500
   agagtgatca aattttgagc tgccttcaat gagccaattt ttgcctataa tggataaagg 4560
   cactttgtct agttcaactg ctcacagaat aatgttaaaa tgaaattaaa acaaggtggg 4620
   ctggtcacac caaaaaaata aaatattaat gtggtgtttg gttagtcgaa tttatcataa 4680
   ttttatagaa attgagacta aaaaacatta agagaataaa ttctataaca aagacaattt 4740
   agtaaaaatg tccttttagg taattttaag tactcttaac caaaataaaa aattcaaatc 4800
   aaattaacca aataagataa tataacatac ggaacatccc acttataatc ttacatcccc 4860
   gtaatcttat tatgaaaagt aatcttatat tactcgaatc aaatgctctc acaaactatt 4920
   atctaaataa agaaaaacac ttaattttta taacattttt tttcatatat ttgaaagatt 4980
   atattttgta tttttacgta aaaatatttg acatagattg agcacctttt taacataatt 5040
   ccaccataag tcaattatgt agatgagaaa ttggtacaaa caacgtggag ccaaatccca 5100
   ccaaaccatc tctcatcctc tcctataaaa ggcttgctac acatagacaa caatccacac 5160
   aaaaatacac ttaaaattct tttctttcta tttggttaac catgtgcgga attgttggtg 5220
   ctatcgccca aagagacgtt gctgagattt tgttagaggg tctgcgaagg ctagagtata 5280
   gaggatatga ctccgctggt ctggctgtcg ttgatgctga gggtcatatg acaaggctaa 5340
   gaaggttagg aaaggttcag atgcttgctc aggcagctga ggaacatcca ttgcatggag 5400
   gtactggtat tgcacatacc aggtgggcta ctcatgggga gccatcagaa gttaatgctc 5460
   atccacatgt gagtgagcat atcgttgtag ttcacaatgg gataattgaa aaccacgaac 5520
   cattgaggga agagttaaag gcaagaggat atacttttgt gagtgagact gacactgagg 5580
   ttattgcaca tttagtgaac tgggaactca aacagggggg cacattgcgt gaggctgtgt 5640
   taagagctat tcctcaactt agaggtgcat acggtactgt tattatggat tcaagacacc 5700
   cagatactct ccttgcagct agatcaggta gtcccttggt cataggactt ggaatgggtg 5760
   aaaattttat cgctagcgac caattggcct tattgccagt tacaagacga tttattttcc 5820
   ttgaagaggg cgatattgct gagattacta gaaggtctgt gaacatcttt gataagactg 5880
   gcgctgaggt taaacgtcag gatatcgagt ctaaccttca atacgatgct ggtgataaag 5940
   gaatttacag gcattatatg caaaaggaaa tttatgaaca accaaatgct atcaaaaaca 6000
   cacttactgg ccgtatttct catggacagg tcgatttaag cgagcttggt cctaatgcag 6060
   acgaactgct atcaaaagtt gagcacatac agatactggc atgcggaact agttataatt 6120
   caggaatggt gtctagatac tggttcgaaa gcttggcagg tataccttgt gatgtagaga 6180
   tcgcttctga gtttaggtat agaaagtctg ctgtgcgtag aaattcatta atgattacat 6240
   tatctcaatc cggagaaaca gcagatacac tggctggatt gaggctttct aaggaactcg 6300
   gatatctggg ttcacttgct atttgtaatg taccaggttc ctcattggtt cgtgaatcag 6360
   atctagcact tatgacaaat gcaggaactg aaataggtgt ggcaagtacc aaggctttca 6420
   caacccaact gaccgtactt ttaatgttgg tagcaaaact cagtcgatta aaggggctag 6480
   atgcatctat cgaacatgat attgttcacg ggcttcaagc tctcccttca agaattgaac 6540
   aaatgctttc acaagataag agaatagagg cattggctga agatttttcc gacaaacatc 6600
   acgcattgtt tcttggacgt ggcgatcaat atccaattgc attggaagga gctttgaagt 6660
   tgaaagaaat aagttacatt cacgcagaag catatgcagc tggagaactc aagcatggtc 6720
   ctttggcact catcgacgct gacatgcccg tgatcgtagt ggctcctaat aacgaactgc 6780
   tcgaaaagct taaatcaaat atcgaagagg ttcgagctag aggaggtcag ctttacgttt 6840
   tcgctgaaca agatgctgga ttcgtgtcaa gcgataatat gcatataatt gaaatgcctc 6900
   acgttgaaga agtgattgca cctatatttt atacagtccc attgcaactt ctagcttacc 6960
   atgttgcact tattaaagga actgatgttg atcagcctag aaacctagca aaatctgtaa 7020
   cagtcgaata aacgcgtggc gcgcccccga tccgcgtttg tgttttctgg gtttctcact 7080
   taagcgtctg cgttttactt ttgtattggg tttggcgttt agtagtttgc ggtagcgttc 7140
   ttgttatgtg taattacgct tttcttcttt gcttcagcag tttcggttga aatataaatc 7200
   gaatcaagtt tcactttatc agcgttgttt taaattttgg cattaaattg gtgaaaattg 7260
   cttcaatttt gtatctaaat agaagagaca acatgaaatt cgacttttga cctcaaatct 7320
   tcgaacattt atttcctgat ttcacgatgg atgaggataa cgaaagggcg gttcctatgt 7380
   ccgggaaagt tcccgtagaa gacaatgagc aaagctactg aaacgcggac acgacgtcgc 7440
   attggtacgg atatgagtta aaccgactca attcctttat taagacataa accgattttg 7500
   gttaaagtgt aacagtgagc tgatataaaa ccgaaacaaa ccggtacaag tttgattgag 7560
   caacttgatg acaaacttca gaattttggt tattgaatga aaatcatagt ctaatcgtaa 7620
   aaaatgtaca gaagaaaagc tagagcagaa caaagattct atattctggt tccaatttat 7680
   catcgcttta acgtccctca gatttgatcg gggaattcga tatcattacc ctgttatccc 7740
   taaagcttat taatgtttgt cgaggagaaa tatgagtcga ggcatggata cactaagttc 7800
   ccctgaagtg agcatgatct ttgatgctga gatgattccc agagcaagat agtttgtgct 7860
   gcaagtgaca caattgtaat gaaaccacca ctcaacgaat ttacttgtgg ctttgacatg 7920
   tcgtgtgctc tgtttgtatt tgtgagtgcc ggttggtaat tatttttgtt aatgtgattt 7980
   taaaacctct tatgtaaata gttactttat ctattgaagt gtgttcttgt ggtctatagt 8040
   ttctcaaagg gaaattaaaa tgttgacatc ccatttacaa ttgataactt ggtatacaca 8100
   aactttgtaa atttggtgat atttatggtc gaaagaaggc aatacccatt gtatgttcca 8160
   atatcaatat caatacgata acttgataat actaacatat gattgtcatt gtttttccag 8220
   tatcaatata cattaagcta ctacaaaatt agtataaatc actatattat aaatcttttt 8280
   cggttgtaac ttgtaattcg tgggttttta aaataaaagc atgtgaaaat tttcaaataa 8340
   tgtgatggcg caattttatt ttccgagttc caaaatattg ccgcttcatt accctaattt 8400
   gtggcgccac atgtaaaaca aaagacgatt cttagtggct atcactgcca tcacgcggat 8460
   cactaatatg aaccgtcgat taaaacagat cgacggttta tacatcattt tattgtacac 8520
   acggatcgat atctcagccg ttagatttaa tatgcgatct gattgctcaa aaaatagact 8580
   ctccgtcttt gcctataaaa acaatttcac atctttctca cccaaatcta ctcttaaccg 8640
   ttcttcttct tctacagaca tcaatttctc tcgactctag aggatccaag cttatcgatt 8700
   tcgaacccct caggcgaaga acaggtatga tttgtttgta attagatcag gggtttaggt 8760
   ctttccatta ctttttaatg ttttttctgt tactgtctcc gcgatctgat tttacgacaa 8820
   tagagtttcg ggttttgtcc cattccagtt tgaaaataaa ggtccgtctt ttaagtttgc 8880
   tggatcgata aacctgtgaa gattgagtct agtcgattta ttggatgatc cattcttcat 8940
   cgtttttttc ttgcttcgaa gttctgtata accagatttg tctgtgtgcg attgtcatta 9000
   cctagccgtg tatcgagaac tagggttttc gagtcaattt tgcccctttt ggttatatct 9060
   ggttcgataa cgattcatct ggattagggt tttaagtggt gacgtttagt attccaattt 9120
   cttcaaaatt tagttatgga taatgaaaat ccccaattga ctgttcaatt tcttgttaaa 9180
   tgcgcagatc acaatggctt cgatctcctc ctcagtcgcg accgttagcc ggaccgcccc 9240
   tgctcaggcc aacatggtgg ctccgttcac cggccttaag tccaacgccg ccttccccac 9300
   caccaagaag gctaacgact tctccaccct tcccagcaac ggtggaagag ttcaatgtat 9360
   gcaggtgtgg ccggcctacg gcaacaagaa gttcgagacg ctgtcgtacc tgccgccgct 9420
   gtctatggcg cccaccgtga tgatggcctc gtcggccacc gccgtcgctc cgttccaggg 9480
   gctcaagtcc accgccagcc tccccgtcgc ccgccgctcc tccagaagcc tcggcaacgt 9540
   cagcaacggc ggaaggatcc ggtgcatggc cggcgccgag gagatcgtgc tgcagcccat 9600
   caaggagatc tccggcaccg tcaagctgcc ggggtccaag tcgctttcca accggatcct 9660
   cctactcgcc gccctgtccg aggggacaac agtggttgat aacctgctga acagtgagga 9720
   tgtccactac atgctcgggg ccttgaggac tcttggtctc tctgtcgaag cggacaaagc 9780
   tgccaaaaga gctgtagttg ttggctgtgg tggaaagttc ccagttgagg atgctaaaga 9840
   ggaagtgcag ctcttcttgg ggaatgctgg aatcgcaatg cggtccttga cagcagctgt 9900
   tactgctgct ggtggaaatg caacttacgt gcttgatgga gtaccaagaa tgagggagag 9960
   acccattggc gacttggttg tcggattgaa gcagcttggt gcagatgttg attgtttcct 10020
   tggcactgac tgcccacctg ttcgtgtcaa tggaatcgga gggctacctg gtggcaaggt 10080
   caagctgtct ggctccatca gcagtcagta cttgagtgcc ttgctgatgg ctgctccttt 10140
   ggctcttggg gatgtggaga ttgaaatcat tgataaatta atctccattc cgtacgtcga 10200
   aatgacattg agattgatgg agcgttttgg tgtgaaagca gagcattctg atagctggga 10260
   cagattctac attaagggag gtcaaaaata caagtcccct aaaaatgcct atgttgaagg 10320
   tgatgcctca agcgcaagct atttcttggc tggtgctgca attactggag ggactgtgac 10380
   tgtggaaggt tgtggcacca ccagtttgca gggtgatgtg aagtttgctg aggtactgga 10440
   gatgatggga gcgaaggtta catggaccga gactagcgta actgttactg gcccaccgcg 10500
   ggagccattt gggaggaaac acctcaaggc gattgatgtc aacatgaaca agatgcctga 10560
   tgtcgccatg actcttgctg tggttgccct ctttgccgat ggcccgacag ccatcagaga 10620
   cgtggcttcc tggagagtaa aggagaccga gaggatggtt gcgatccgga cggagctaac 10680
   caagctggga gcatctgttg aggaagggcc ggactactgc atcatcacgc cgccggagaa 10740
   gctgaacgtg acggcgatcg acacgtacga cgaccacagg atggcgatgg ctttctccct 10800
   tgccgcctgt gccgaggtcc ccgtcaccat ccgggaccct gggtgcaccc ggaagacctt 10860
   ccccgactac ttcgatgtgc tgagcacttt cgtcaagaat taagctctag aactagtgga 10920
   tcccccgatc cgcgtttgtg ttttctgggt ttctcactta agcgtctgcg ttttactttt 10980
   gtattgggtt tggcgtttag tagtttgcgg tagcgttctt gttatgtgta attacgcttt 11040
   ttcttcttgc ttcagcagtt tcggttgaaa tataaatcga atcaagtttc actttatcag 11100
   cgttgtttta aattttggca ttaaattggt gaaaattgct tcaattttgt atctaaatag 11160
   aagagacaac atgaaattcg acttttgacc tcaaatcttc gaacatttat ttcctgattt 11220
   cacgatggat gaggataacg aaagggcggt tcctatgtcc gggaaagttc ccgtagaaga 11280
   caatgagcaa agctactgaa acgcggacac gacgtcgcat tggtacggat atgagttaaa 11340
   ccgactcaat tcctttatta agacataaac cgattttggt taaagtgtaa cagtgagctg 11400
   atataaaacc gaaacaaacc ggtacaagtt tgattgagca acttgatgac aaacttcaga 11460
   attttggtta ttgaatgaaa atcatagtct aatcgtaaaa aatgtacaga agaaaagcta 11520
   gagcagaaca aagattctat attctggttc caatttatca tcgctttaac gtccctcaga 11580
   tttgatcggg aaaccaaaac gtcgtgagac agtttggtta actataacgg tcctaaggta 11640
   gcgatcgagg cattacggca ttacggcact cgcgagggtc cgaattcgag catggagcca 11700
   tttacaattg aatatatcct gccgccgctg ccgctttgca cccggtggag cttgcatgtt 11760
   ggtttctacg cagaactgag ccggttaggc agataatttc cattgagaac tgagccatgt 11820
   gcaccttccc cccaacacgg tgagcgacgg ggcaacggag tgatccacat gggactttta 11880
   aacatcatcc gtcggatggc gttgcgagag aagcagtcga tccgtgagat cagccgacgc 11940
   accgggcagg cgcgcaacac gatcgcaaag tatttgaacg caggtacaat cgagccgacg 12000
   ttcacggtac cggaacgacc aagcaagcta gcttagtaaa gccctcgcta gattttaatg 12060
   cggatgttgc gattacttcg ccaactattg cgataacaag aaaaagccag cctttcatga 12120
   tatatctccc aatttgtgta gggcttatta tgcacgctta aaaataataa aagcagactt 12180
   gacctgatag tttggctgtg agcaattatg tgcttagtgc atctaacgct tgagttaagc 12240
   cgcgccgcga agcggcgtcg gcttgaacga attgttagac attatttgcc gactaccttg 12300
   gtgatctcgc ctttcacgta gtggacaaat tcttccaact gatctgcgcg cgaggccaag 12360
   cgatcttctt cttgtccaag ataagcctgt ctagcttcaa gtatgacggg ctgatactgg 12420
   gccggcaggc gctccattgc ccagtcggca gcgacatcct tcggcgcgat tttgccggtt 12480
   actgcgctgt accaaatgcg ggacaacgta agcactacat ttcgctcatc gccagcccag 12540
   tcgggcggcg agttccatag cgttaaggtt tcatttagcg cctcaaatag atcctgttca 12600
   ggaaccggat caaagagttc ctccgccgct ggacctacca aggcaacgct atgttctctt 12660
   gcttttgtca gcaagatagc cagatcaatg tcgatcgtgg ctggctcgaa gatacctgca 12720
   agaatgtcat tgcgctgcca ttctccaaat tgcagttcgc gcttagctgg ataacgccac 12780
   ggaatgatgt cgtcgtgcac aacaatggtg acttctacag cgcggagaat ctcgctctct 12840
   ccaggggaag ccgaagtttc caaaaggtcg ttgatcaaag ctcgccgcgt tgtttcatca 12900
   agccttacgg tcaccgtaac cagcaaatca atatcactgt gtggcttcag gccgccatcc 12960
   actgcggagc cgtacaaatg tacggccagc aacgtcggtt cgagatggcg ctcgatgacg 13020
   ccaactacct ctgatagttg agtcgatact tcggcgatca ccgcttccct catgatgttt 13080
   aactttgttt tagggcgact gccctgctgc gtaacatcgt tgctgctcca taacatcaaa 13140
   catcgaccca cggcgtaacg cgcttgctgc ttggatgccc gaggcataga ctgtacccca 13200
   aaaaaacagt cataacaagc catgaaaacc gccactgcgc cgttaccacc gctgcgttcg 13260
   gtcaaggttc tggaccagtt gcgtgagcgc atacgctact tgcattacag cttacgaacc 13320
   gaacaggctt atgtccactg ggttcgtgcc ttcatccgtt tccacggtgt gcgtcacccg 13380
   gcaaccttgg gcagcagcga agtcgaggca tttctgtcct ggctggcgaa cgagcgcaag 13440
   gtttcggtct ccacgcatcg tcaggcattg gcggccttgc tgttcttcta cggcaagtgc 13500
   tgtgcacgga tctgccctgg cttcaggaga tcggaagacc tcggccgtcc gggcgcttgc 13560
   cggtggtgct gaccccggat gaagtctcta gagctctaga gggttcgcat cctcggtttt 13620
   ctggaaggcg agcatcgttt gttcgcccag cttctgtatg gaacgggcat gcggatcagt 13680
   gagggtttgc aactgcgggt caaggatctg gatttcgatc acggcacgat catcgtgcgg 13740
   gagggcaagg gctccaagga tcgggccttg atgttacccg agagcttggc acccagcctg 13800
   cgcgagcagg gatcgatacc gtgcggctgc atgaaatcct ggccggtttg tctgatgcca 13860
   agctggcggc ctggccggcc agcttggccg ctgaagaaac cgagcgccgc cgtctaaaaa 13920
   ggtgatgtgt atttgagtaa aacagcttgc gtcatgcggt cgctgcgtat atgatgcgat 13980
   gagtaaataa acaaatacgc aaggggaacg catgaaggtt atcgctgtac ttaaccagaa 14040
   aggcgggtca ggcaagacga ccatcgcaac ccatctagcc cgcgccctgc aactcgccgg 14100
   ggccgatgtt ctgttagtcg attccgatcc ccagggcagt gcccgcgatt gggcggccgt 14160
   gcgggaagat caaccgctaa ccgttgtcgg catcgaccgc ccgacgattg accgcgacgt 14220
   gaaggccatc ggccggcgcg acttcgtagt gatcgacgga gcgccccagg cggcggactt 14280
   ggctgtgtcc gcgatcaagg cagccgactt cgtgctgatt ccggtgcagc caagccctta 14340
   cgacatatgg gccaccgccg acctggtgga gctggttaag cagcgcattg aggtcacgga 14400
   tggaaggcta caagcggcct ttgtcgtgtc gcgggcgatc aaaggcacgc gcatcggcgg 14460
   tgaggttgcc gaggcgctgg ccgggtacga gctgcccatt cttgagtccc gtatcacgca 14520
   gcgcgtgagc tacccaggca ctgccgccgc cggcacaacc gttcttgaat cagaacccga 14580
   gggcgacgct gcccgcgagg tccaggcgct ggccgctgaa attaaatcaa aactcatttg 14640
   agttaatgag gtaaagagaa aatgagcaaa agcacaaaca cgctaagtgc cggccgtccg 14700
   agcgcacgca gcagcaaggc tgcaacgttg gccagcctgg cagacacgcc agccatgaag 14760
   cgggtcaact ttcagttgcc ggcggaggat cacaccaagc tgaagatgta cgcggtacgc 14820
   caaggcaaga ccattaccga gctgctatct gaatacatcg cgcagctacc agagtaaatg 14880
   agcaaatgaa taaatgagta gatgaatttt agcggctaaa ggaggcggca tggaaaatca 14940
   agaacaacca ggcaccgacg ccgtggaatg ccccatgtgt ggaggaacgg gcggttggcc 15000
   aggcgtaagc ggctgggttg tctgccggcc ctgcaatggc actggaaccc ccaagcccga 15060
   ggaatcggcg tgacggtcgc aaaccatccg gcccggtaca aatcggcgcg gcgctgggtg 15120
   atgacctggt ggagaagttg aaggccgcgc aggccgccca gcggcaacgc atcgaggcag 15180
   aagcacgccc cggtgaatcg tggcaagcgg ccgctgatcg aatccgcaaa gaatcccggc 15240
   aaccgccggc agccggtgcg ccgtcgatta ggaagccgcc caagggcgac gagcaaccag 15300
   attttttcgt tccgatgctc tatgacgtgg gcacccgcga tagtcgcagc atcatggacg 15360
   tggccgtttt ccgtctgtcg aagcgtgacc gacgagctgg cgaggtgatc cgctacgagc 15420
   ttccagacgg gcacgtagag gtttccgcag ggccggccgg catggccagt gtgtgggatt 15480
   acgacctggt actgatggcg gtttcccatc taaccgaatc catgaaccga taccgggaag 15540
   ggaagggaga caagcccggc cgcgtgttcc gtccacacgt tgcggacgta ctcaagttct 15600
   gccggcgagc cgatggcgga aagcagaaag acgacctggt agaaacctgc attcggttaa 15660
   acaccacgca cgttgccatg cagcgtacga agaaggccaa gaacggccgc ctggtgacgg 15720
   tatccgaggg tgaagccttg attagccgct acaagatcgt aaagagcgaa accgggcggc 15780
   cggagtacat cgagatcgag ctagctgatt ggatgtaccg cgagatcaca gaaggcaaga 15840
   acccggacgt gctgacggtt caccccgatt actttttgat cgatcccggc atcggccgtt 15900
   ttctctaccg cctggcacgc cgcgccgcag gcaaggcaga agccagatgg ttgttcaaga 15960
   cgatctacga acgcagtggc agcgccggag agttcaagaa gttctgtttc accgtgcgca 16020
   agctgatcgg gtcaaatgac ctgccggagt acgatttgaa ggaggaggcg gggcaggctg 16080
   gcccgatcct agtcatgcgc taccgcaacc tgatcgaggg cgaagcatcc gccggttcct 16140
   aatgtacgga gcagatgcta gggcaaattg ccctagcagg ggaaaaaggt cgaaaaggtc 16200
   tctttcctgt ggatagcacg tacattggga acccaaagcc gtacattggg aaccggaacc 16260
   cgtacattgg gaacccaaag ccgtacattg ggaaccggtc acacatgtaa gtgactgata 16320
   taaaagagaa aaaaggcgat ttttccgcct aaaactcttt aaaacttatt aaaactctta 16380
   aaacccgcct ggcctgtgca taactgtctg gccagcgcac agccgaagag ctgcaaaaag 16440
   cgcctaccct tcggtcgctg cgctccctac gccccgccgc ttcgcgtcgg cctatcgcgg 16500
   ccgctggccg ctcaaaaatg gctggcctac ggccaggcaa tctaccaggg cgcggacaag 16560
   ccgcgccgtc gccactcgac cgccggcgcc cacatcaagg caccctgcct cgcgcgtttc 16620
   ggtgatgacg gtgaaaacct ctgacacatg cagctcccgg agacggtcac agcttgtctg 16680
   taagcggatg ccgggagcag acaagcccgt cagggcgcgt cagcgggtgt tggcgggtgt 16740
   cggggcgcag ccatgaccca gtcacgtagc gatagcggag tgtatactgg cttaactatg 16800
   cggcatcaga gcagattgta ctgagagtgc accatatgcg gtgtgaaata ccgcacagat 16860
   gcgtaaggag aaaataccgc atcaggcgct cttccgcttc ctcgctcact gactcgctgc 16920
   gctcggtcgt tcggctgcgg cgagcggtat cagctcactc aaaggcggta atacggttat 16980
   ccacagaatc aggggataac gcaggaaaga acatgtgagc aaaaggccag caaaaggcca 17040
   ggaaccgtaa aaaggccgcg ttgctggcgt ttttccatag gctccgcccc cctgacgagc 17100
   atcacaaaaa tcgacgctca agtcagaggt ggcgaaaccc gacaggacta taaagatacc 17160
   aggcgtttcc ccctggaagc tccctcgtgc gctctcctgt tccgaccctg ccgcttaccg 17220
   gatacctgtc cgcctttctc ccttcgggaa gcgtggcgct ttctcatagc tcacgctgta 17280
   ggtatctcag ttcggtgtag gtcgttcgct ccaagctggg ctgtgtgcac gaaccccccg 17340
   ttcagcccga ccgctgcgcc ttatccggta actatcgtct tgagtccaac ccggtaagac 17400
   acgacttatc gccactggca gcagccactg gtaacaggat tagcagagcg aggtatgtag 17460
   gcggtgctac agagttcttg aagtggtggc ctaactacgg ctacactaga aggacagtat 17520
   ttggtatctg cgctctgctg aagccagtta ccttcggaaa aagagttggt agctcttgat    17580
   ccggcaaaca aaccaccgct ggtagcggtg gtttttttgt ttgcaagcag cagattacgc    17640
   gcagaaaaaa aggatctcaa gaagatccgg aaaacgcaag cgcaaagaga aagcaggtag    17700
   cttgcagtgg gcttacatgg cgatagctag actgggcggt tttatggaca gcaagcgaac    17760
   cggaattgcc agattcgaag ctcggtcccg tgggtgttct gtcgtctcgt tgtacaacga    17820
   aatccattcc cattccgcgc tcaagatggc ttcccctcgg cagttcatca gggctaaatc    17880
   aatctagccg acttgtccgg tgaaatgggc tgcactccaa cagaaacaat caaacaaaca    17940
   tacacagcga cttattcaca cgcgaca                                         17967